# EUROPEAN PATENT APPLICATION

(11) **EP 2 085 016 A1**
(43) Date of publication of application: **05.08.2009**
(21) Application number: 07832036.3
(22) Date of filing: 16.11.2007
(51) Int. Cl.: A61B 1/00

(54) **MEDICAL DEVICE**

(30) Priority: 22.11.2006 JP 2006315721; 04.12.2006 JP 2006327417; 11.12.2006 JP 2006333533
(71) Applicant: Olympus Medical Systems Corp., Tokyo 151-0072 (JP)
(72) Inventor: MATSUURA, Nobuyuki, Tokyo 192-8512 (JP); MATSUI, Raifu, Tokyo 192-8512 (JP)
(74) Representative: von Hellfeld, Axel
(86) International application number: PCT/JP2007/072306
(87) International publication number: WO 2008/062733

(57) **Abstract**

The present invention relates to a medical apparatus including a hollow member with inner surface parts configured to be brought into contact with each other through deformation of the hollow member and is directed to provide a medical apparatus wherein inner surface parts of a hollow member are prevented from being adhered to each other. A medical apparatus includes a hollow member (60) whose inner surface parts are configured to be brought into contact with each other through deformation of the hollow member (60) and the hollow member (60) includes a convex and concave portion (70) formed on the inner surface parts configured to be brought into contact with each other.

## Description

### Technical Field

The present invention relates to a medical apparatus including a hollow member with inner surface parts configured to be brought into contact with each other through deformation of the hollow member.

### Background Art

In a medical apparatus, various hollow members are used whose inner surface parts are configured to be bought into contact with each other through deformation thereof. As the hollow member, there is a fluid transferring tube disclosed in Jpn. Pat. Appln. KOKAI Publication No. 8-308786. The fluid transferring tube is configured to be closed when it is pressed in a valve and opened when the pressing is released. Moreover, as the hollow member, there is a balloon of an insertion instrument for an endoscope disclosed in Jpn. Pat. Appln. KOKAI Publication No. 2002-301019. The insertion instrument for the endoscope is adapted to assist an insertion of the endoscope into a cavity in the body. An insertion instrument main part has a shape of a sheath, and the endoscope is inserted through the inner cavity of the insertion instrument main part so as to be movable forward and backward. The balloon is provided on the outside of the distal end portion of the insertion instrument main part, and it is possible for the balloon to hold the inner surface of the cavity in the body by expanding the balloon.

### Disclosure of Invention

In the fluid transferring tube disclosed in Jpn. Pat. Appln. KOKAI Publication No. 8-308786, the inner surface parts of the tube are brought into contact with each other when the tube is closed by the valve. In the balloon of the insertion instrument for the endoscope disclosed in the Jpn. Pat. Appln. KOKAI Publication No. 2002-301019, the inner surface parts of the balloon are brought into contact with each other when the balloon is contracted. In the case where the inner surface parts are kept in contact with each other for a long time, there is a possibility that the inner surface parts are adhered to each other.

The present invention has been made in view of the above mentioned problem and is directed to provide a medical apparatus wherein inner surface parts of a hollow member are prevented from being adhered to each other.

In an aspect of the present invention, a medical apparatus is characterized by including a hollow member whose inner surface parts are configured to be brought into contact with each other through deformation of the hollow member, and the hollow member includes a convex and concave portion formed on the inner surface parts configured to be brought into contact with each other.

In this medical apparatus, the convex and concave portion is formed on the inner surface parts of the hollow member, and therefore, the contact area of the inner surface pats is reduced, whereby preventing the inner surface parts from being adhered.

In a preferred aspect of the present invention, the medical apparatus is **characterized in that** the hollow member is formed of an elastic member.

In this medical apparatus, the convex and concave portion is applied to the elastic member whose inner surface parts tend to be in contact with each other through deformation thereof, and therefore, the adhesion preventing effect is remarkably exhibited.

In a preferred aspect of the present invention, the medical apparatus is **characterized in that** the elastic member is made of silicon, polyurethane, or vinyl chloride.

In this medical apparatus, the convex and concave portion is applied to the elastic member made of silicon, polyurethane or vinyl chloride which tends to adhere, and therefore, the adhesion preventing effect is particularly remarkably exhibited.

In a preferred aspect of the present invention, the medical apparatus is **characterized in that** the hollow member is a balloon configured to be expanded and contracted.

In this medical apparatus, the convex and concave portion is applied to the balloon which tends to adhere because the inner surface parts thereof are brought into contact with each other when contracted, and therefore, the adhesion preventing effect is remarkably exhibited.

In a preferred aspect of the present invention, the medical apparatus is **characterized in that** the hollow member is a fluid transferring tube.

In this medical apparatus, the convex and concave portion is applied to the fluid transferring tube which tends to adhere because the fluid transferring tube is soft and has a small diameter, and is configured to be essily closed to bring the inner surface pats thereof into contact with each other, and therefore, the adhesion preventing effect is remarkably exhibited.

In a preferred aspect of the present invention, the medical apparatus is **characterized in that** the fluid transferring tube is configured to be closed and opened by a valve configure to press the fluid transferring tube from an outside.

In this medical apparatus, the convex and concave portion is applied to the fluid transferring tube which tends to adhere because the fluid transferring tube is configured to be pressed from the outside by the valve to press the inner surface parts thereof to each other, and therefore, the adhesion preventing effect is particularly remarkably exhibited.

In a preferred aspect of the present invention, the medical apparatus is **characterized in that** the fluid transferring tube is housed within the medical apparatus.

In this medical apparatus, the convex and concave portion is applied to the fluid transferring tube within the medical apparatus in order to prevent adhesion, which it is troublesome to exchange in the case of adhesion, and therefore, it is easy to maintain the medical apparatus.

In a preferred aspect of the present invention, the medical apparatus is **characterized in that** a contamination generating source is located on an upstream side and the fluid transferring tube is provided on more downstream side than a contamination removing mechanism with respect to the contamination generating source.

In this medical apparatus, the contamination generating source is located on the upstream side, and the fluid transferring tube is provided on the more downstream side than the contamination removing mechanism with respect to the contamination generating source, and therefore, it is possible to reduce the number of exchange of the fluid transferring tube due to pollution by contamination and to inexpensively maintain the medical apparatus.

In a preferred aspect of the present invention, the medical apparatus is **characterized in that** the hollow member is an insertion instrument main part for an endoscope including an inner cavity through which an insertion portion of an endoscope is inserted so as to be movable forward and backward.

In this medical apparatus, the inner surface parts of the insertion instrument main part for the endoscope is prevented from being adhered to each other, and also, the convex and concave portion reduces contact area between the inner surface of the insertion instrument for the endoscope and the outer surface of the endoscope, whereby reducing insertion resistance of the endoscope to the insertion instrument for the endoscope.

In a preferred aspect of the present invention, the medical apparatus is characterized by including: the insertion instrument main part having a tubular shape and including a distal end portion and a proximal end portion; a liquid transferring pass extending from the proximal end portion of the insertion instrument main part toward a distal end side and configured to transfer a liquid; a gas transferring pass extending from the proximal end portion of the insertion instrument main part toward the distal end side and configured to transfer a gas; a liquid connecter protruding from the proximal end portion of the insertion instrument main part, forming a proximal end portion of the liquid transferring pass, configured to be connected to a liquid transferring apparatus, and whose protruding direction and a longitudinal direction of the insertion instrument main part toward a distal end side forms a first inclination angle; and a gas connecter protruding from the proximal end portion of the insertion instrument main part, forming a proximal end portion of the gas transferring pass, configured to be connected to a gas transferring apparatus, and whose protruding direction and the longitudinal direction of the insertion instrument main part toward the distal end side forms a second inclination angle, and the second inclination angle is smaller than the first inclination angle.

In this medical apparatus, the inclination angle of the liquid connecter is relatively large, and therefore, the length of the liquid transferring pass extending from the protruding end portion of the liquid connecter to the root end portion of the liquid connecter is short, the total length of the liquid transferring pass is short, and conduit resistance over the whole liquid transferring pass is small. Moreover, the inclination angle of the gas connecter is relatively small, and therefore, a bending part of the gas transferring pass formed from the root end portion of the gas connecter to the insertion instrument main part is gentle, conduit resistance in the bending part is small, and conduit resistance over the whole gas transferring pass is small. Therefore, it is possible to transfer a fluid at small resistance.

In a preferred aspect of the present invention, the medical apparatus is **characterized in that** an inner diameter of the liquid transferring pass is larger than an inner diameter of the gas transferring pass.

In this medical apparatus, regarding a liquid having relatively high viscosity and large resistance in transfer in comparison with a gas, the inner diameter of the liquid transferring pass is large and conduit resistance thereof is small, and therefore, it is possible to transfer a liquid at small resistance.

In a preferred aspect of the present invention, the medical apparatus is **characterized in that** the liquid transferring pass is configured to transfer a lubricant.

In this medical apparatus, a lubricant having high viscosity and large resistance in transfer is transferred through the liquid transferring pass, and therefore, the resistance reducing effect is remarkably exhibited.

In a preferred aspect of the present invention, the medical apparatus is **characterized in that** the liquid transferring pass is a liquid supplying pass configured to supply a liquid.

In this medical apparatus, the inclination angle of the liquid connecter is large, and therefore, when inserting the endoscope through the insertion instrument and then supplying a liquid to the liquid connecter of the insertion instrument, regarding a force applying to the liquid connecter, a component force in the longitudinal direction of the insertion instrument is small, and the insertion instrument is prevented from being displaced with respect to the endoscope.

In a preferred aspect of the present invention, the medical apparatus is **characterized in that** a gas tube is to be connected to the gas connecter and the gas connecter and a gas transferring apparatus are to be connected to each other through the gas tube.

In this medical apparatus, the inclination angle of the gas connecter is small, and therefore, when connecting the gas tube to the gas connecter, an inclination angle of the gas tube become small, and the insertion instrument and the gas tube has an integrated form along the longitudinal direction of the insertion instrument, whereby preventing the gas tube from hampering operation.

In a preferred aspect of the present invention, the medical apparatus is characterized by including: other gas transferring pass extending from the proximal end portion of the insertion instrument main part to more distal end side of the insertion instrument main part than the gas transferring pass and configured to transfer a gas; and other gas connecter protruding from the proximal end portion of the insertion instrument main part, forming a proximal end portion of the other gas transferring pass, configured to be connected to a gas transferring apparatus and whose protruding direction and the longitudinal direction of the insertion instrument main part toward the distal end side forms other second inclination angle, and wherein the other second inclination angle is smaller than the second inclination angle.

In this medical apparatus, regarding the gas transferring pass extending to more distal end side, having longer total length, and so having larger conduit resistance among the gas transferring passes, the inclination angle of the corresponding gas connecter is smaller and the bending part is gentler, and therefore, the inclination angles of the gas connecters are optimally set.

In a preferred aspect of the present invention, the medical apparatus is characterized by including: an endoscope including an insertion portion provided on a distal end side of the endoscope and configured to be inserted into a cavity in a body and an endoscope engagement portion provided in a proximal end portion of the endoscope; an insertion instrument including the insertion instrument main part with a distal end portion and a proximal end portion and an insertion instrument engagement portion provided in the proximal end portion of the insertion instrument main part and configured to be engaged with the endoscope engagement portion.

In this medical apparatus, it is possible to sufficiently fix the endoscope and the insertion instrument to each other by engaging the endoscope engagement portion and the insertion instrument engagement portion with each other, and also, the insertion portion engagement portion scarcely hampers an insertion when inserting the insertion portion of the endoscope into the insertion instrument, and it is possible to smoothly insert the insertion portion of the endoscope into the insertion instrument.

In a preferred aspect of the present invention, the medical apparatus is **characterized in that** the endoscope engagement portion and the insertion instrument engagement portion include an endoscope fitting portion and an insertion instrument fitting portion configured to be fitted to each other, respectively.

In this medical apparatus, it is possible to securely fix the endoscope and the insertion instrument to each other by fitting the endoscope fitting portion and the insertion instrument fitting portion to each other.

In a preferred aspect of the present invention, the medical apparatus is **characterized in that** the endoscope includes a bending preventing portion provided on more proximal end side than the insertion portion and whose outer peripheral surface has a tapering shape, the endoscope fitting portion is formed of the outer peripheral surface of the bending preventing portion, and the insertion portion fitting portion includes a tapering portion configured to be fitted to the outer peripheral surface of the bending preventing portion.

In this medical apparatus, the endoscope fitting portion is formed of the outer peripheral surface of the bending preventing portion generally used in the endoscope, and therefore, it is unnecessary to additionally processing the endoscope, and it is possible to inexpensively embody.

In a preferred aspect of the present invention, the medical apparatus is **characterized in that** one fitting portion of the endoscope fitting portion and the insertion instrument fitting portion includes a fitting convex portion, and the other fitting portion of the endoscope fitting portion and the insertion instrument fitting portion includes a fitting concave portion configured to be fitted to the fitting convex portion.

In this medical apparatus, it is possible to sufficiently securely fix the endoscope and the insertion instrument to each other by fitting the fitting concave portion and the fitting convex portion to each other.

In a preferred aspect of the present invention, the medical apparatus is **characterized in that** one engagement portion of the endoscope engagement portion and the insertion instrument engagement portion includes a pin, the other engagement portion of the endoscope engagement portion and the insertion instrument engagement portion includes an engagement groove portion in which the pin is configured to be slid, and the engagement groove portion includes a holding portion configured to hold the pin such that the endoscope is unmovable forward and backward with respect to the insertion instrument and a guide portion connecting to the holding portion and configured to guide the pin.

In this medical apparatus, it is possible to securely fix the endoscope to the insertion instrument so as to unmovable forward and backward by holding the pin in the holding portion of the engagement groove portion, and also, it is possible to easily fix and release the insertion instrument and the endoscope to and from each other by moving the endoscope with respect to the insertion instrument and guiding the pin to and from the holding portion through the guide portion of the engagement groove portion.

In a preferred aspect of the present invention, the medical apparatus is **characterized in that** at least one fitting portion of the endoscope fitting portion and the insertion instrument fitting portion includes an adhesion preventing mechanism configured to prevent adhesion between the endoscope fitting portion and the insertion instrument fitting portion.

In this medical apparatus, the adhesion preventing mechanism prevents the endoscope fitting portion and the insertion instrument fitting portion from being adhered to each other, and therefore, it is prevented that releasing the fixing between the insertion instrument and the endoscope is made impossible.

In a preferred aspect of the present invention, the medical apparatus is **characterized in that** the adhesion preventing mechanism includes an adhesion preventing concave portion formed on a fitting surface of the at least one fitting portion of the endoscope fitting portion and the insertion instrument fitting portion.

In this medical apparatus, the adhesion preventing concave portion formed on the fitting surface prevents the endoscope fitting portion and the insertion instrument fitting portion from being adhered to each other.

In a preferred aspect of the present invention, the medical apparatus is **characterized in that** at least one fitting portion of the endoscope fitting portion and the insertion instrument fitting portion includes an adhesion releasing mechanism configured to release adhesion between the endoscope fitting portion and the insertion instrument fitting portion.

In this medical apparatus, the adhesion releasing mechanism enables to release adhesion between the endoscope fitting portion and the insertion instrument fitting portion, and therefore, it is possible to easily release the fixing between the endoscope and the insertion instrument even when both the fitting portions are adhered to each other.

In a preferred aspect of the present invention, the medical apparatus is **characterized in that** the adhesion releasing mechanism is formed of a breaking mechanism configured to break the at least one fitting portion of the endoscope fitting portion and the insertion instrument fitting portion.

In this medical apparatus, adhesion between both the fitting portions are released by breaking the fitting portion.

In a preferred aspect of the present invention, the medical apparatus is **characterized in that** the adhesion releasing mechanism is formed of a deformation mechanism configured to deform at least one fitting portion of the endoscope fitting portion and the insertion instrument fitting portion to separate a fitting surface of the endoscope fitting portion and a fitting surface of the insertion instrument fitting portion from each other.

In this medical apparatus, adhesion between both the fitting portions is released by deforming the fitting portion to separate the fitting surfaces of both the fitting portions from each other.

### Brief Description of Drawings

FIG. 1 is a view showing a medical apparatus according to a first embodiment of the present invention;
FIG. 2 is a view showing an endoscope and an insertion instrument according to the first embodiment of the present invention;
FIG. 3A is a view showing the inner surface of a balloon according to the first embodiment of the present invention;
FIG. 3B is a longitudinal cross sectional view showing the balloon according to the first embodiment of the present invention;
FIG. 4A is a view showing the inner surface of the balloon according to a first variation example of the first embodiment of the present invention;
FIG. 4B is a longitudinal cross sectional view showing the balloon according to the first variation example of the first embodiment of the present invention;
FIG. 5A is a view showing the inner surface of a balloon according to a second variation example of the first embodiment of the present invention;
FIG. 5B is a longitudinal cross sectional view showing the balloon according to the second variation example of the first embodiment of the present invention;
FIG. 6A is a view showing the inner surface of a balloon according to a third variation example of the first embodiment of the present invention;
FIG. 6B is a transverse cross sectional view showing the balloon according to the third variation example of the first embodiment of the present invention;
FIG. 7 is a view showing the inner surface of a balloon according to a fourth variation example of the first embodiment of the present invention;
FIG. 8 is a schematic view showing a method for producing a balloon according to the first embodiment of the present invention;
FIG. 9A is a perspective view showing the balloon in an expanding state according to the first embodiment of the present invention;
FIG. 9B is a perspective view showing the balloon in a contracting state according to the first embodiment of the present invention;
FIG. 9C is a transverse cross sectional view showing the balloon in the contracting state according to the first embodiment of the present invention;
FIG. 10 is a perspective view showing a tube according to the first embodiment of the present invention;
FIG. 11 is a transparent perspective view showing a tube according to a variation example of the first embodiment of the present invention;
FIG. 12A is a cross sectional view showing a electromagnetic valve in the state where the electromagnetic valve closes an air discharging pass according to the first embodiment of the present invention;
FIG. 12B is a cross sectional view showing the electromagnetic valve in the state where the electromagnetic valve closes an air supplying pass according to the first embodiment of the present invention;
FIG. 13 is a longitudinal cross sectional view showing a balloon according to a third embodiment of the present invention;
FIG. 14 is a longitudinal cross sectional view showing a balloon according to a variation example of the third embodiment of the present invention;
FIG. 15A is a schematic view showing a supplying and discharging apparatus according to a fourth embodiment of the present invention;
FIG. 15B is a schematic view showing a supplying and discharging apparatus according to a variation example of the fourth embodiment of the present invention;
FIG. 16 is a schematic view showing a supplying and discharging apparatus according to a fifth embodiment of the present invention;
FIG. 17 is a partially cross sectional side view showing an insertion instrument according to a sixth embodiment of the present invention;
FIG. 18A is a partially cross sectional side view showing a proximal end portion of an insertion instrument according to the sixth embodiment of the present invention;
FIG. 18B is a partially cross sectional side view showing a proximal end portion of an insertion instrument according to a comparison embodiment with the sixth embodiment of the present invention;
FIG. 19 is a partially cross sectional side view showing an insertion instrument according to a first variation example of the sixth embodiment of the present invention;
FIG. 20 is a perspective view showing an insertion instrument according to a second variation example of the sixth embodiment of the present invention;
FIG. 21 is a partially cross sectional side view showing an insertion instrument according to a seventh embodiment of the present invention;
FIG. 22A is a longitudinal cross sectional view showing a distal end portion of an insertion instrument according to a reference embodiment of the present invention;
FIG. 22B is a front view showing the distal end portion of the insertion instrument according to a reference embodiment of the present invention;
FIG. 23 is partially cross sectional side view showing an endoscope and an insertion instrument according to an eighth embodiment of the present invention;
FIG. 24 is a partially cross sectional side view showing an endoscope and an insertion instrument according to a ninth embodiment of the present invention;
FIG. 25 is a partially cross sectional side view showing an endoscope and an insertion instrument according to a tenth embodiment of the present invention;
FIG. 26 is a side view showing an endoscope and an insertion instrument according to an eleventh embodiment of the present invention;
FIG. 27 is a perspective view showing a proximal end portion of an insertion instrument according to a twelfth embodiment of the present invention;
FIG. 28 is a perspective view showing a proximal end portion of an insertion instrument according to a thirteenth embodiment of the present invention; and
FIG. 29 is a perspective view showing a proximal end portion of an insertion instrument according to a fourteenth embodiment of the present invention.

Best Mode for Carrying Out the Invention Hereinafter, a first to a fourth embodiment of the present invention will be described referring to the drawings.

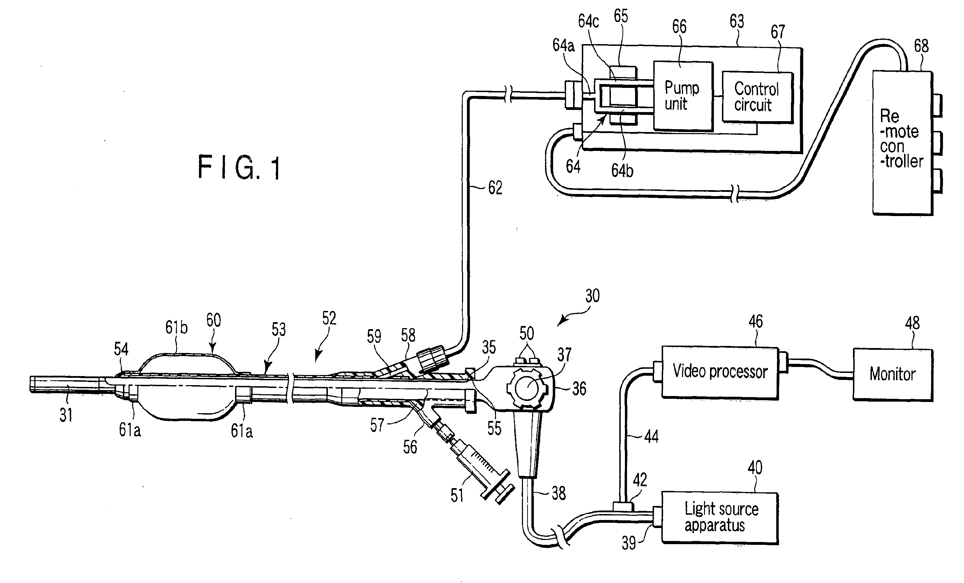

FIGS. 1 to 12B show the first embodiment of the present invention.

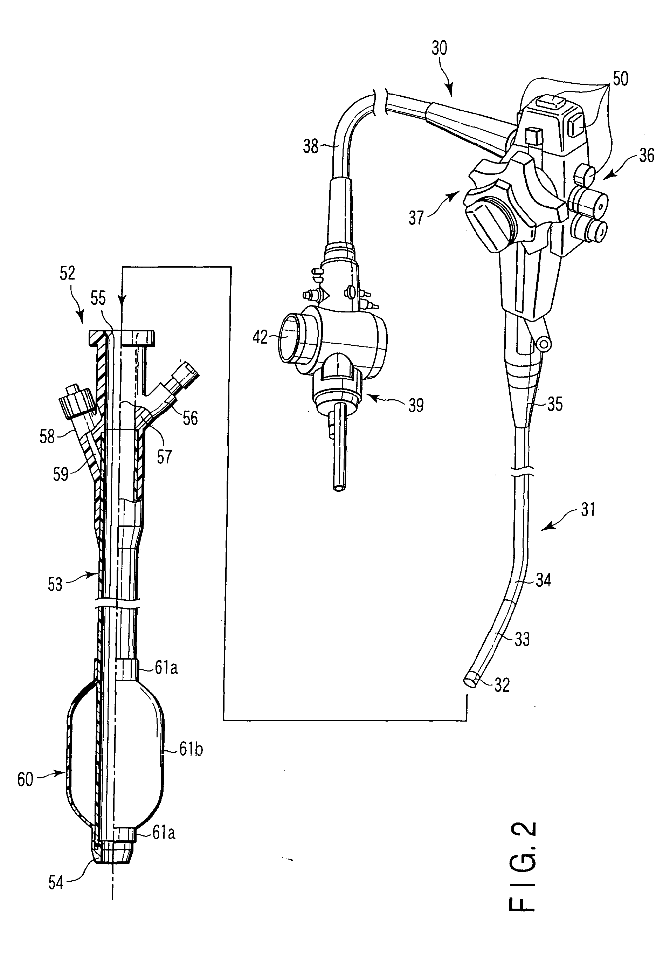

Referring to FIGS. 1 and 2, an endoscope 30 of an endoscope apparatus according to the present embodiment includes an elongate insertion portion 31 configured to be inserted into a cavity in the body. The insertion portion 31 is formed of a distal end rigid portion 32 having rigidity, a bending portion 33 configured to be operated to be bent and an insertion tube portion 34 being long and flexible coupled to each other in the order from the distal end side. The proximal end portion of the insertion portion 31 is coupled to an operation portion 36 through a bending preventing portion 35, and the operation portion 36 is configured to be held and operated by an operator. The bending preventing portion 35 is configured to prevent the insertion portion 31 from bending in the coupling part between the insertion portion 31 and the operation portion 36, and has a tapering shape whose outer diameter is reduced from the proximal end side to the distal end side. The operation portion 36 is provided with a bending operation knob to operate the bending portion 33, and the like. A universal cable 38 extends from the operation portion 36, and the extended end portion of the universal cable 38 is provided with a light source connecter 39 and an electric connecter 42. The light source connecter 39 is configured to be connected to a light source apparatus 40, and illumination light from the light source apparatus 40 is to be transmitted through a light guide extending from the light source connecter 39 to the distal end portion of the endoscope 30 and emitted from the distal end portion of the endoscope 30. The electric connecter 42 is configured to be connected to a video processor 46 through an electric cable 44, and an image signal obtained in an image pick-up unit in the distal end portion of the endoscope 30 is output to the video processor 46 through a signal cable and the electric cable 44 extending from the distal end portion of the endoscope 30 to the electric connecter 42. The video processor 46 is configured to process the input image signal and displays an observation image in a monitor 48. The operation portion 36 of the endoscope 30 is provided with various kinds of switch 50 to operate the video processor 46.

On the other hand, an insertion instrument 52 of the endoscope apparatus according to the present embodiment includes an insertion instrument main part 53 being soft and having a shape of a sheath. The insertion portion 31 of the endoscope 30 is to be inserted through the inner cavity of the insertion instrument main part 53 from the proximal end opening to the distal end opening so as to be movable forward and backward. The distal end portion of the insertion instrument main part 53 is provided with a distal end cap 54. The distal end portion of the insertion portion 31, which is consisted of the distal end rigid portion 32 and the bending portion 33, is to be projected from the distal end opening of the insertion instrument main part 53. A tapering portion 55 is formed in the proximal end portion of the inner cavity of the insertion instrument main part 53, and the inner diameter of the tapering portion 55 is reduced from the proximal end side to the distal end side. When the insertion portion 31 and then the bending preventing portion 35 of the endoscope 30 are inserted into the proximal end opening of the insertion instrument main part 53 and the tapering portion 55 of the insertion instrument main part 53 and the bending preventing portion 35 of the endoscope 30 are fitted to each other, the insertion instrument 52 and the endoscope 30 are fixed to each other.

The proximal end portion of the insertion instrument main part 53 is provided with a liquid connecter 56, and a liquid supplying pass 57 as a liquid transferring pass extends from the liquid connecter 56 to the inner cavity of the insertion instrument main part 53. A cylinder 51 and so on as a liquid transfer apparatus is configured to supply a lubricant from the liquid connecter 56 through the liquid supplying pass 57 to the inner cavity of the insertion instrument main part 53. The lubricant is adapted to improve a sliding capability between the inner peripheral surface of the insertion instrument 52 and the outer peripheral surface of the insertion portion 31.

The proximal end portion of the insertion instrument main part 53 is provided with the gas connecter 58, a gas supplying and discharging pass 59 as a gas transferring pass extends from the gas connecter 58 to a balloon 60 as a hollow member, and the balloon 60 is provided on the outside of the distal end portion of the insertion instrument main part 53. The balloon 60 has a substantially cylindrical shape, and both the end parts 61a of the balloon 60 is bonded and fixed to the outer peripheral surface of the insertion instrument main part 53. The middle part 61b of the balloon 60 is configured to be expanded and contracted by supplying and discharging a gas from and to the gas connecter 58 through the gas supplying and discharging pass 59 to and from the balloon 60.

One end portion of an outer tube 62 as a fluid transferring tube and a gas tube is detachably connected to the gas connecter 58, and the other end portion of the outer tube 62 is detachably connected to a supplying and discharging apparatus 63 as a gas transferring apparatus. An inner tube 64 as a fluid transferring tube is provided within the supplying and discharging apparatus 63. The outer tube 62 and the inner tube 64 are made of material having elasticity, for example, silicon, polyurethane, or vinyl chloride. One end side of the inner tube 64 forms a common pass 64a and is fluidly communicated to the outer tube 62. On the other hand, the other end side of the inner tube 64 is branched into an air supplying pass 64b and an air discharging pass 64c. The air supplying pass 64b and the air discharging pass 64c are configured to be selectively opened and closed by an electromagnetic valve 65. The air supplying pass 64b is connected to an air supplying pump of a pump unit 66 and the air discharging pass 64c is connected to an air discharging pump of the pump unit 66. The electromagnetic valve 65 and the pump unit 66 are controlled by a control circuit 67, and a remote controller 68 is connected to the supplying and discharging apparatus 63 and configured to input an operation signal to the control circuit 67.

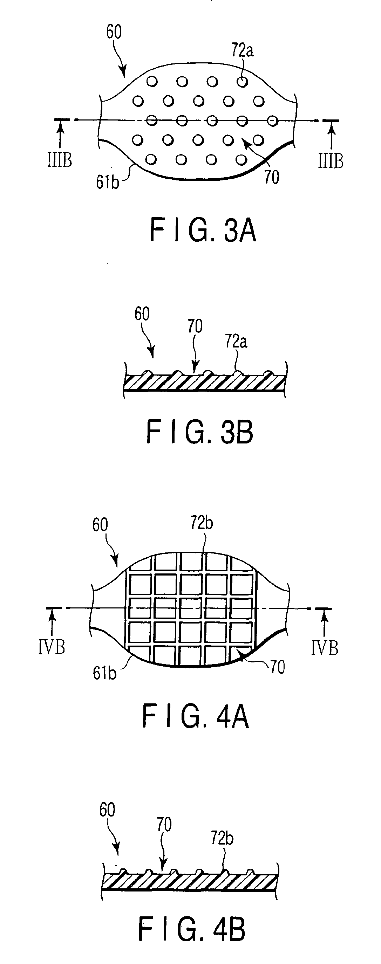

Referring to FIGS. 3A to 8, the balloon 60 provided on the outside of the distal end portion of the insertion instrument main part 53 will be explained in detail.

A convex and concave portion 70 is formed on the inner surface of the middle part 61b of the balloon 60 configured to be expanded and contracted. When the balloon 60 is contracted and the inner surface parts of the balloon 60 are brought into contact with each other, the convex and concave portion 70 reduces contact area to prevent adhesion between the inner surface parts of the balloon 60. The convex and concave shape of the convex and concave portion 70 may be any shape adapted to reduce the contact area between the inner surface parts of the balloon 60. Hereinafter, examples of the convex and concave shape will be described.

In a balloon 6Q as shown in FIGS. 3A and 3B, a large number of semi-spherical convex shapes 72a are formed on the inner surface of the middle part 61b of the balloon 60.

In a balloon 60 as shown in FIGS. 4A and 4B, elongate convex shapes 72b are formed into a grid pattern on the inner surface of the middle part 61b of the balloon 60.

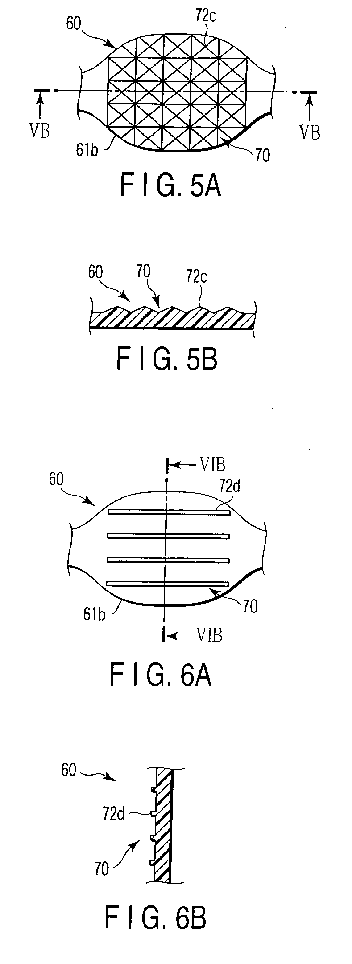

In a balloon 60 as shown in FIGS. 5A and 5B, regular quadrangular pyramid-like convex shapes 72c are arranged side by side longitudinally and transversely on the inner surface of the middle part 61b of the balloon 60.

In a balloon 60 as shown in FIGS. 6A and 6B, elongate convex shapes 72d are arranged side by side and extend in the longitudinal direction on the inner surface of the middle part 61b of the balloon 60.

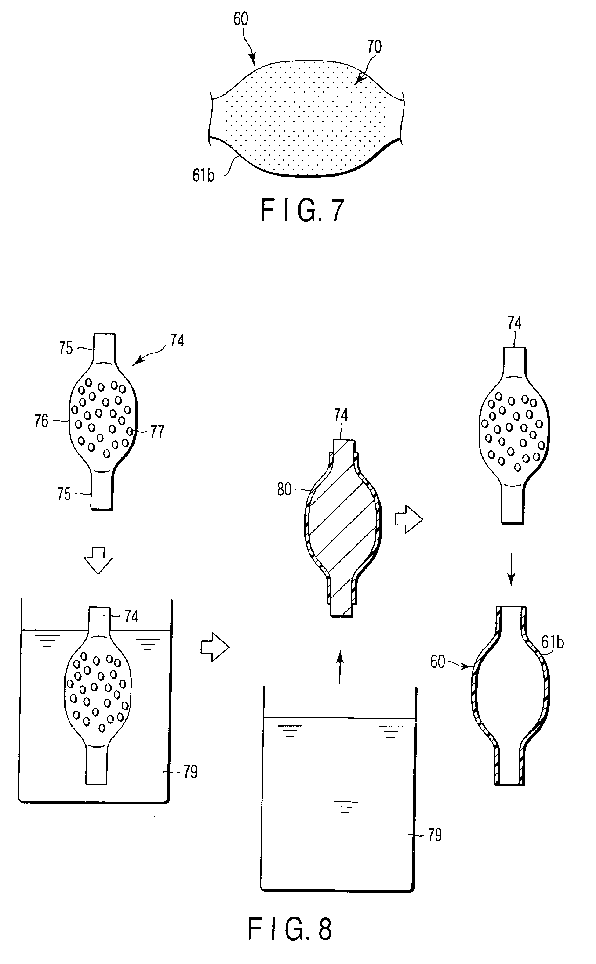

In a balloon 60 as shown in FIG. 7, a pear-skin-like convex and concave portion 70 is formed on the inner surface of the middle part 61b of the balloon 60.

Referring to FIG. 8, a method for producing a balloon 60 will be explained.

A case will be explained as an example, where a balloon 60 as shown in FIG. 3A and 3B is produced through dip molding. A die 74 is prepared, whose shape of the outer surface corresponds to the shape of the inner surface of the balloon 60. That is, the die 74 is formed of both circularly columnar portions 75 on both end parts thereof and an expanding portion 76 whose outer diameter is increased on a middle part thereof. In the expanding portion 76, a large number of semi-spherical concave shapes 77 corresponding to the convex shapes 72a of the balloon 60 are formed, and the convex and concave shape obtained by reversing the convex and concave shape of the balloon 60 is formed. The die 74 is dipped into liquid silicon rubber 79 and then taken out, and the liquid silicon rubber 80 attached to the outer surface of the die 74 is dried. After that, the die 74 is taken from the dried silicon rubber, and the balloon 60 is formed where the convex and concave shape is transferred to the inner surface of the middle part 61b of the balloon 60. Other than such dip molding, the convex and concave shape may be transferred to the inner surface of the balloon 60 through pressing.

Next, an adhesion preventing function of the balloon 60 will be explained.

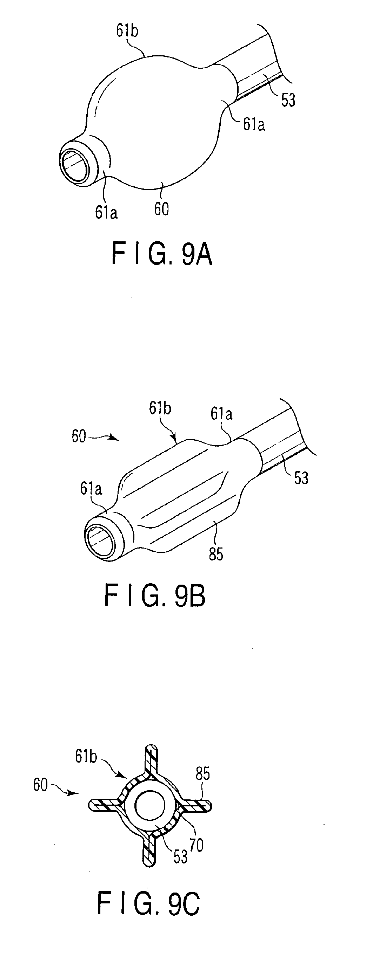

As is shown in FIG. 9A, when the balloon 60 is expanded, the inner surface parts of the balloon 60 are separated from each other. On the other hand, as is shown in FIG. 9B and 9C, when the balloon 60 is contracted, the outer wall parts of the balloon 60 are overlapped with each other to form overlaped parts 85, the overlaped parts 85 are arranged side by side in the peripheral direction and extend in the longitudinal direction, and the inner surface parts of the balloon 60 are brought into contact with each other in the overlapping parts 85. Therefore, there is a possibility that the inner surface parts of the balloon 60 are adhered to each other. In particular, the balloon 60 is kept contracted for a long time while the medical apparatus is not used, the inner surface parts of the balloon 60 are kept in contact with each other for a long time, and there is a high possibility of adhesion. In the balloon 60 according to the present embodiment, the convex and concave portion 70 is formed on the inner surface parts configured to be brought into contact with each other when the balloon 60 is contracted, and therefore, contact area of the inner surface parts is reduced, whereby preventing adhesion.

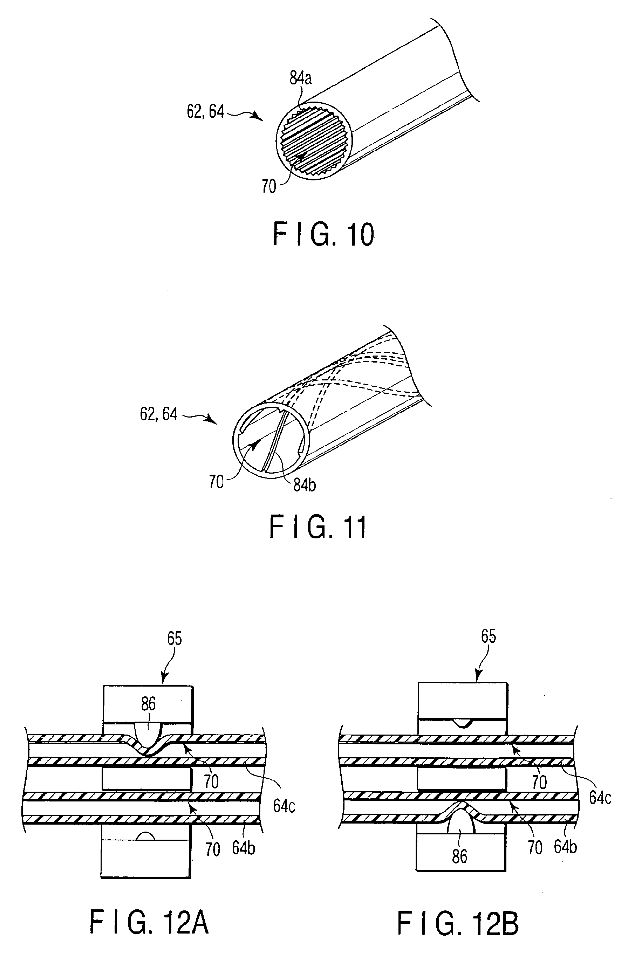

Referring to FIG. 10 and 11, the outer tube 62 and the inner tube 64 will be explained in detail.

A convex and concave portion 70 is formed on the inner surface of the tube 62 or 64. The convex and concave portion 70 is configured to reduce contact area to prevent the inner surface parts of the tube 62 or 64 from being adhered to each other when the tube 62 or 64 is closed and the inner surface parts of the tube 62 or 64 are brought into contact with each other. The convex and concave shape of the convex and concave portion 70 may be any shape adapted to reduce contact area between the inner surface parts of the tube 62 or 64. Hereinafter, examples of the convex and concave shape will be described.

In a tube 62 or 64 as shown in FIG. 10, a large number of elongate convex shapes 84a extend in the longitudinal direction and arranged side by side in the peripheral direction.

In a tube 62 or 64 as shown in FIG. 11, elongate convex shapes 84b helically extend in the longitudinal direction.

Next, an adhesion preventing function of the outer tube 62 or the inner tube 64 will be explained.

The outer tube 62 is located outside, and therefore, the outer tube 62 may be bent or crushed to be closed and the inner surface parts of the outer tube 62 may be brought into contact with each other. Therefore, there is a possibility that the inner surface parts of the outer tube 62 are adhered to each other. In particular, in the case where the outer tube 62 is kept in storage in the state where the outer tube 62 is bent or crushed while the medical apparatus is not used, the inner surface parts of the outer tube 62 is kept in contact with each other for a long time, and therefore, there is a high possibility of adhesion. In the outer tube 62 according to the present embodiment, the convex and concave portion 70 is formed on the inner surface parts configured to be brought into contact with each other when the outer tube 62 is closed, and therefore, contact area between the inner surface parts is reduced, whereby preventing adhesion.

As is shown in FIG. 12A, when expanding the balloon 60, in the supplying and discharging apparatus 63, a pressing portion 86 of the electromagnetic valve 65 presses the air discharging pass 64c of the inner tube 64 to be closed, while the air supplying pass 64b of the inner tube 64 is kept opened. On the other hand, as is shown in FIG. 12B, when contracting the balloon 60, in an supplying and discharging apparatus 63, the pressing portion 86 of the electromagnetic valve 65 presses the air supplying pass 64b of the inner tube 64 to be closed, while the air discharging pass 64c of the inner tube 64 is kept opened.

Here, when the air discharging pass 64c of the inner tube 64 is closed, the inner surface parts of the inner tube 64 are brought into contact with each other in the air discharging pass 64c, and also, when the air supplying pass 64b of the inner tube 64 is closed, the inner surface parts of the inner tube 64 are brought into contact with each other in the air supplying pass 64b. Therefore, there is a possibility that the inner surface parts of the inner tube 64 are adhered to each other. In particular, the air supplying pass 64b is closed to contract the balloon 60 at the end of using of the medical apparatus, and therefore, while the medical apparatus is not used, the air supplying pass 64b is kept closed and the inner surface parts of the inner tube 64 are in contact with each other in the air supplying pass 64b for a long time. Therefore, there is a high possibility of adhesion. In the inner tube 64 according to the present embodiment, the convex and concave portion 70 is formed on the inner surface parts configured to be brought into contact with each other when the inner tube 64 is closed, and therefore, contact area between the inner surface parts is reduced, whereby preventing adhesion.

The medical apparatus according to the present embodiment exhibits the following effects.

In the medical apparatus according to the present embodiment, the convex and concave portion 70 formed on the inner surface parts of the balloon 60, the outer tube 62 and the inner tube 64 reduces contact area between the inner surface parts, whereby preventing adhesion between the inner surface parts.

In particular, the balloon 60 tends to adhere because the balloon 60 is made of elastic material which tends to adhere, for example, silicon, polyurethane or vinyl chloride and also the inner surface parts of the balloon 60 are brought into contact with each other when the balloon 60 is contracted. The outer tube 62 tends to adhere because the outer tube 62 is made of material which tends to adhere as is similar to the balloon 60 and also the outer tube 62 is soft and has a small diameter, and is configured to be easily closed to bring the inner surface parts of the outer tube 62 in contact with each other. The inner tube 64 tends to adhere because the inner tube 64 is made of material which tends to adhere as is similar to the balloon 60 and the outer tube 62 and also the inner surface parts of the inner tube 64 is configured to be pressed to each other by the electromagnetic valve 65. The convex and concave portion 70 is applied to such balloon 60, outer tube 62 and inner tube 64, and therefore, the adhesion preventing effect is remarkably exhibited.

Moreover, the convex and concave portion 70 is applied to the inner tube 64 within the supplying and discharging apparatus 63 to prevent adhere, which it is troublesome to exchange in the case of adhesion, and therefore, it is easy to maintain the supplying and discharging apparatus 63.

Referring again to FIGS. 1 and 2, the second embodiment of the present invention will be explained.

In a medical apparatus according to the present embodiment, a convex and concave portion 70 is formed over the whole inner surface of an insertion instrument main part 53 as a hollow member. The insertion instrument main part 53 is elongate and soft, and therefore, the insertion instrument main part 53 may be bent or crushed to be closed to bring the inner surface parts thereof into contact with each other to be adhered. In particular, in the case where the insertion instrument main part 53 is kept in storage in the state where the insertion instrument main part 53 is bent or crushed while the medical apparatus is not used, the inner surface parts of the insertion instrument main part 53 is kept in contact with each other for a long time, and therefore, it is a high possibility of adhesion. In the present embodiment, the convex and concave portion 70 is formed on the inner surface parts of the insertion instrument main part 53 configured to be brought into contact with each other when the insertion instrument main part 53 is closed, and therefore, contact area between the inner surface parts is reduced, whereby preventing adhesion. In addition, when inserting an insertion portion 31 of an endoscope 30 through the insertion instrument main part 53 and moving forward and backward the insertion portion 31, the convex and concave portion 70 of the inner surface of the insertion instrument main part 53 reduces contact area and therefore friction resistance between the inner surface of the insertion instrument main part 53 and the outer surface of the insertion portion 31, and therefore, it is possible to smoothly move forward and backward the insertion portion 31.

Moreover, in the case where the insertion portion 31 and then a bending preventing portion 35 of the endoscope 30 is inserted into the proximal end opening of the insertion instrument main part 53 and the bending preventing portion 35 is fitted to a tapering portion 55 of the proximal end portion of the insertion instrument main part 53, the inner surface of the tapering portion 55 and the outer surface of the bending preventing portion 35 is kept pressed, and therefore, there is a possibility of adhesion. In particular, in the case where an insertion instrument 52 and the endoscope 30 is kept in standby for a long time in the state where the insertion instrument 52 and the endoscope 30 are fixed to each other, the inner surface of the tapering portion 55 and the outer surface of the bending preventing portion 35 is kept pressed for a long time, and therefore, there is a high possibility of adhesion. In the present embodiment, the convex and concave portion 70 is formed on the tapering portion 55 at the proximal end portion of the insertion instrument main part 53, and therefore, contact area between the inner surface of the tapering portion 55 and the outer surface of the bending preventing portion 35 is reduced, whereby preventing adhesion.

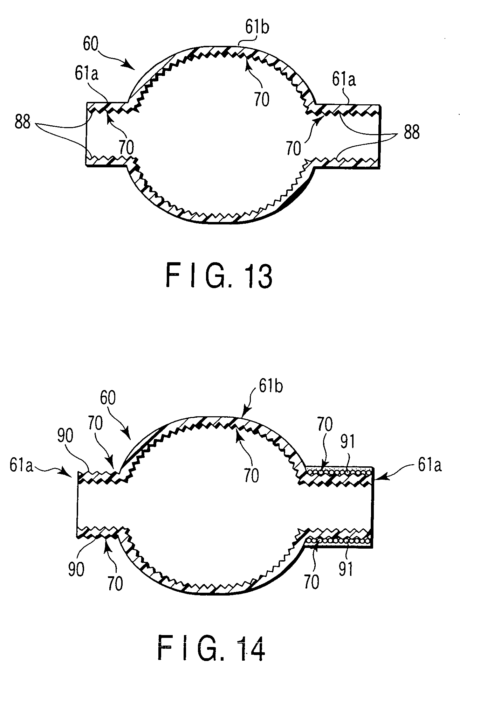

FIG. 13 shows a third embodiment of the present invention.

As is mentioned above, adhesion between the inner surface parts is prevented in the case where a convex and concave portion 70 is formed on the inner surface parts configured to be brought into contact with each other, on the other hand, a bonding effect is improved in the case where the convex and concave portion 70 is formed on a surface to be bonded. Referring to FIGS. 1, 2 and 13, in a balloon 60 according to the present embodiment, the convex and concave portion 70 is also formed on the inner surface 88 of both end parts 61a configured to be bound to be fixed to an insertion instrument main part 53 as well as the inner surface of a middle part 61b configured to be expanded and contracted, whereby improving a bonding effect between the insertion instrument main part 53 and the balloon 60.

FIG. 14 shows a variation example of the third embodiment of the present invention.

Referring to FIGS. 1, 2 and 14, in a balloon 60 according to the present embodiment, both end parts 61a of a balloon 60 are fitted on the outside of the insertion instrument main part 53, silk gut 91 is wound around and fixed to by bonding agent the outer peripheral surface of both the end parts 61a of the balloon 60, and both the end portions of the balloon 60 are fixed to the insertion instrument main part 53. Here, a convex and concave portion 70 is formed on the outer peripheral surface 90 of both the end portions of the balloon 60, whereby improving a bonding effect between the balloon 60 and the silk gut 91.

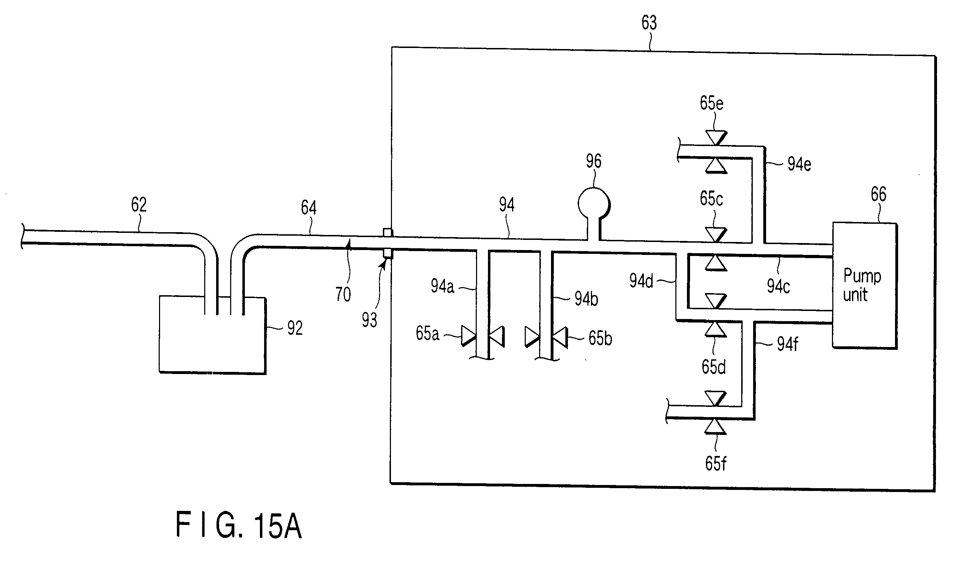

FIG. 15A shows a fourth embodiment of the present invention.

In a medical apparatus according to the present embodiment, a convex and concave portion 70 is not formed on the inner surface of an outer tube 62, differing from the first embodiment. The outer tube 62 is connected to an inner tube 64 through a liquid saving tank 92 adapted to collect a contamination as a contamination removing mechanism. The liquid saving tank 92 may be replaced by a filter adapted to separate a gas and a liquid. The inner tube 64 is put out of the liquid saving tank 92 and put into a supplying and discharging apparatus 63 through a connecting portion 93. In the supplying and discharging apparatus 63, a main pass 94 of the inner tube 64 branches into a first branch pass 94a and further branches into a second branch pass 94b. A pressure sensor 96 is interposed into the main pass 94 more downstream than the second branch pass 94b. Furthermore, the main pass 94 branches into a third branch pass 94c and a fourth branch pass 94d. The third branch pass 94c is connected to a pump unit 66, and a fifth branch pass 94e is branched from the third branch pass 94c on the upper side of the pump unit 66. On the other hand, the fourth branch pass 94d is connected to the pump unit 66, and the sixth branch pass 94f is branched from the fourth branch pass 94d on the upper side of the pump unit 66. A first to a sixth electromagnetic valve 65a, 65b, 65c, 65d, 65e, 65f is interposed into the first to the sixth branch pass 94a ... 94f, respectively.

The medical apparatus according to the present embodiment, even when the supplying and discharging apparatus 63 suctions a contamination by mistake, the liquid saving tank 92 collects the contamination, and therefore, the contamination is prevented from entering into the inner tube 64. Therefore, the inner tube 64 is not contaminated and it is enough to exchange the only outer tube 62. That is, it is unnecessary to frequently exchange the inner tube 64 whose inner surface is provided with the convex and concave portion 70 and which is relatively expensive in comparison with the outer tube 62 whose inner surface is not provided with the convex and concave portion 70 and which is relatively inexpensive, and it is possible to inexpensively maintain the medical apparatus.

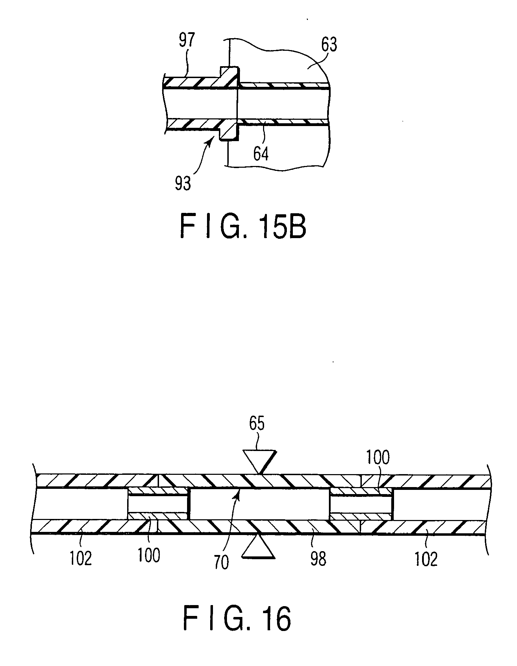

As shown in FIG. 15B, a liquid saving tank 92 and a supplying and discharging apparatus 63 may be connected to each other through a connecting tube 97, and the one end portion of the connecting tube 97 may be configured to be attached to and detached from a connecting portion 93 to the supplying and discharging apparatus 63. Here, a convex and concave portion 70 is not formed on the inner surface of the connecting tube 97, and the convex and concave portion 70 is formed on the only inner surface of the inner tube 64 within the supplying and discharging apparatus 63.

FIG. 16 shows a fifth embodiment of the present invention.

In a supplying and discharging apparatus 63 according to the present embodiment, a convex and concave portion 70 is formed on the inner surface of a processed tube 98, and the processed tube 98 is used in an only part configured to be closed by the electromagnetic valve 65. Both the end portions of the processed tube 98 are connected through the coupling tube 100 to normal tubes 102 whose inner surface is not provided with the convex and concave portion 70, respectively. Instead of using the coupling tube 100, the processed tube 98 and the normal tube 102 may be bonded or welded to each other.

In the present embodiment, the processed tube 98 whose inner surface is provided with the convex and concave portion 70 and which is relatively expensive is used in the only part configured to be closed by the electromagnetic valve 65, the normal tubes 102 whose inner surface are not provided with the convex and concave portion 70 and which are relatively inexpensive are used in the other parts, and therefore, it is possible to inexpensively form the whole medical apparatus.

Hereinafter, a sixth and a seventh embodiment of the present invention, and a reference embodiment thereof will be explained referring to the drawings.

The sixth and the seventh embodiment relates to an insertion instrument adapted to assist an insertion of the endoscope into a cavity in the body.

When an insertion portion of an endoscope is inserted into a cavity in the body, an insertion instrument adapted to assist the insertion of the insertion portion is used.

In Jpn. Pat. Appln. KOKAI Publication No. 2004-329720, an overtube as the insertion instrument is disclosed. The insertion portion of the endoscope is inserted through the overtube so as to be movable forward and backward. When inserting the insertion portion into the overtube, water as a lubricant is injected into the inner cavity of the overtube from a water injecting inlet on the proximal end portion of the overtube whereby improving a sliding capability between the inner surface of the overtube and the outer surface of the insertion portion to improve an inserting capability. Then, in the state where the insertion portion is inserted through the overtube, the overtube and the insertion portion are inserted into a cavity in the body, and then, the overtube and the insertion portion are alternately moved forward to be inserted into a deep portion of the cavity in the body. If necessary, air is supplied to and discharged from a balloon provided on the distal end portion of the overtube through an air supplying tube extending in the longitudinal direction of the overtube from and to an air sending inlet provided on the proximal end portion of the overtube, and therefore, the balloon is expanded to hold the inner surface of the cavity in the body and contracted to release the holding.

In the insertion instrument disclosed in Jpn. Pat. Appln. KOKAI Publication No. 2004-329720, water is to be supplied into the inner cavity from the water injection inlet, and also, air is to be supplied to and discharged from the balloon from and to the air sending inlet. In order to smoothly and easily carry out the transfer of the fluid, it is preferable that resistance in the transfer of the fluid is small.

The sixth and the seventh embodiment have been made in view of the above mentioned problem and are directed to provide an insertion instrument wherein it is possible to transfer a fluid at small resistance.

An endoscope apparatus and an insertion instrument according to the sixth and the seventh embodiment, and the reference embodiment thereof include substantially similar structures to those of the endoscope apparatus according to the first embodiment as shown in FIGS. 1 and 2, and the insertion instrument according to the second embodiment explained referring to FIGS. 1 and 2. Hereinafter, configurations differing from the first and the second embodiment will be explained in details.

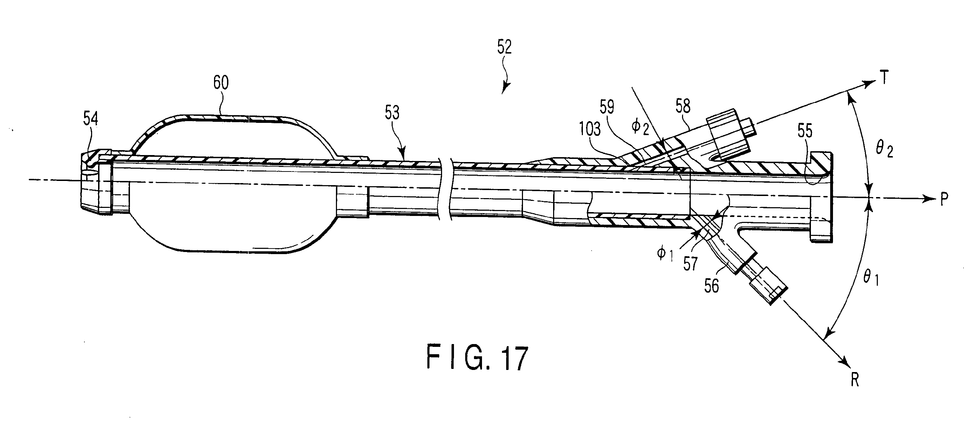

FIGS. 17 to 18B show the sixth embodiment of the present invention.

Referring to FIG. 17, a liquid connecter 56 and a gas connecter 58 will be explained in detail.

A protruding direction R of the liquid connecter 56 forms a first inclination angle θ₁ with respect to the longitudinal direction P of an insertion instrument main part 53 of the insertion instrument 52 toward the distal end side. On the other hand, a protruding direction T of the gas connecter 58 forms a second inclination angle θ₂ with respect to the longitudinal direction P of the insertion instrument main part 53 toward the distal end side. The second inclination angle θ₂ of the gas connecter 58 is smaller than the first inclination angle θ₁ of the liquid connecter 56. That is, regarding a liquid supplying pass 57, the inclination angle θ₁ of the liquid connecter 56 is relatively large, the total length of the liquid supplying pass 57 having a shape of a substantially straight line and extending from the protruding end of the liquid connecter 56 to the inner cavity of the insertion instrument main part 53 is short, and conduit resistance over the whole liquid supplying pass 57 is small. On the other hand, a gas supplying and discharging pass 59 extends along the longitudinal direction (the protruding direction) of the gas connecter 58, and then, extends along the longitudinal direction of the insertion instrument main part 53 to reach a balloon 60. That is, the gas supplying and discharging pass 59 forms a bending part 103 from the root end portion of the gas connecter 58 to the insertion instrument main part 53. Here, the inclination angle θ₂ of the gas connecter 58 is relatively small, and therefore, the bending part 103 is gentle, and conduit resistance over the whole gas supplying and discharging pass 59 is small.

Furthermore, the inner diameter ϕ₁ of the liquid supplying pass 57 is larger than the inner diameter ϕ₂ of the gas supplying and discharging pass 59. That is, regarding the liquid supplying pass 57, the inner diameter ϕ₁ is relatively large, conduit resistance over the whole liquid supplying pass 57 is further small.

Next, a method for using the insertion instrument according to the present embodiment will be explained.

When inserting an endoscope 30 into a cavity in the body, an insertion portion 31 of the endoscope 30 is inserted into a proximal end opening of the insertion instrument main part 53, and then, while a lubricant is supplied from the liquid connecter 56 through the liquid supplying pass 57 to the inner cavity of the insertion instrument main part 53 of the insertion instrument 52 using a cylinder 51 and so on, the insertion portion 31 is inserted through the insertion instrument main part 53. The lubricant supplied to the inner cavity of the insertion instrument main part 53 is carried by the insertion portion 31 to be spread to the distal end side following the insertion of the insertion portion 31, and the lubricant improves a sliding capability between the inner peripheral surface of the insertion instrument 52 and the outer peripheral surface of the insertion portion 31.

Here, as is mentioned above, the conduit resistance of the liquid supplying pass 57 is small, and therefore, resistance in supplying the lubricant through the liquid supplying pass 57 is sufficiently small. Hydrochloric acid lidocaine jelly or glycerol jelly which has large viscosity is used as the lubricant, and therefore, the resistance reducing effect is remarkably exhibited.

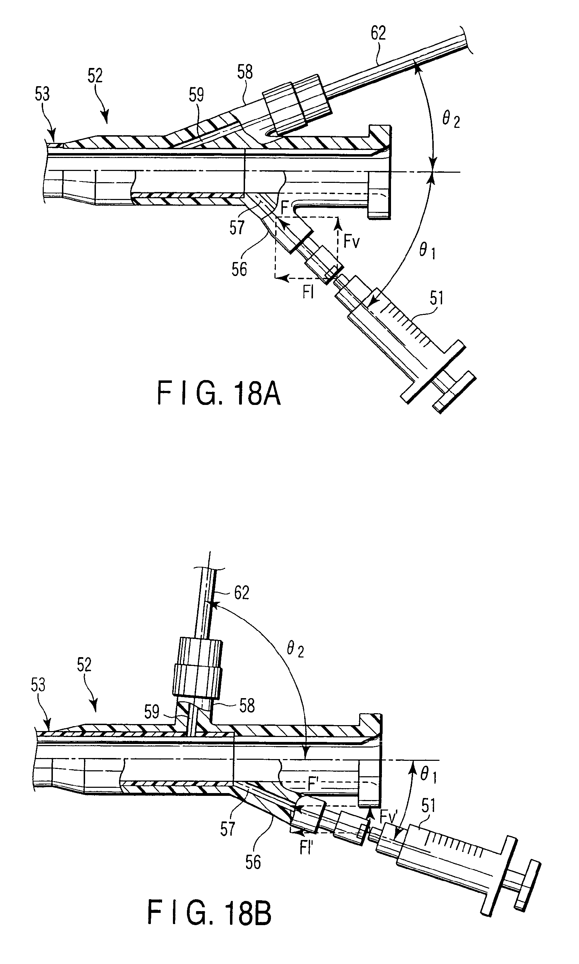

Moreover, referring to FIGS. 18A and 18B, when supplying the lubricant by the cylinder 51, force F, F' is applied to the liquid connecter 56 in a direction along the liquid supplying pass 57, that is, in the longitudinal direction of the liquid connecter 56 toward the insertion instrument main part 53. The force F, F' may be resolved into component force Fl, F1' in the longitudinal direction of the insertion instrument main part 53 and component force Fv, Fv' in a direction perpendicular to the longitudinal direction. The component force Fl, Fl' in the longitudinal direction of the insertion instrument main part 53 is to push forward the insertion instrument 52 with respect to the endoscope 30 and is to function to displace the insertion instrument 52 with respect to the endoscope 30. Here, as shown in FIG. 18B, in the case where an inclination angle θ₂ of the gas connecter 58 is larger than an inclination angle θ₁ of the liquid connecter 56, the inclination angle θ₁ of the liquid connecter 56 is relatively small, the component force Fl' in the longitudinal direction of the insertion instrument main part 53 is large, and therefore, the insertion instrument 52 is easily displaced with respect to the endoscope 30 due to supplying of the lubricant. In the present embodiment, as shown in FIG. 18A, the inclination angle θ₁ of the liquid connecter 56 is relatively large, the component force Fl in the longitudinal direction of the insertion instrument main part 53 is small, and therefore, it is prevented that the insertion instrument 52 is displaced with respect to the endoscope 30 due to supplying of the lubricant.

After the insertion instrument 52 and the endoscope 30 is inserted into the cavity in the body together with each other, the insertion instrument 52 and the endoscope 30 is alternately moved forward to be inserted into a deep portion of the cavity in the body.

If necessary, a supplying and discharging apparatus 63 is operated through the remote controller 68, air is supplied through an outer tube 62, a gas connecter 58 and a gas supplying and discharging pass 59 to the balloon 60 to expand the balloon 60 to hold the inner surface of the body wall. Moreover, air is discharged from the balloon 60 through the gas supplying and discharging pass 59, the gas connecter 58 and the outer tube 62 to contract the balloon 60 to release the holding to the inner surface of the body wall. Here, as is mentioned above, the conduit resistance of the gas supplying and discharging pass 59 is small, and therefore, resistance in supplying and discharging air through the gas supplying and discharging pass 59 is sufficiently small.

Furthermore, referring to FIGS. 18A and 18B, the outer tube 62 connected to the gas connecter 58 extends toward the proximal end side to the supplying and discharging apparatus 63 and is configured to be moved following movement of the insertion instrument 52, and therefore, the outer tube 62 may hamper an operation of the insertion instrument 52 and the endoscope 30. As shown in FIG. 18B, in the case where an inclination angle θ₂ of the gas connecter 58 is larger than an inclination angle θ₁ of the liquid connecter 56, the inclination angle θ₂ of the gas connecter 58 is relatively large, an inclination angle of the outer tube 62 is large, and therefore, the outer tube 62 tends to hamper an operation. In the present embodiment, as shown in FIG. 18A, the inclination angle θ₂ of the gas connecter 58 is relatively small, and therefore, an inclination angle of the outer tube 62 is small and the insertion instrument 52 and the outer tube 62 has an integrated form along the longitudinal direction of the insertion instrument 52, whereby preventing the outer tube 62 from hampering an operation.

As is mentioned above, in the insertion instrument 52 according to the present embodiment, the inclination angle θ₁ of the liquid connecter 56 is relatively large, and therefore, the length of the liquid supplying pass 57 extending from the protruding end of the liquid connecter 56 to the inner cavity of the insertion instrument main part 53 is short, the total length of the liquid supplying pass 57 is short, and the conduit resistance over the whole liquid supplying pass 57 is small. Moreover, the inclination angle θ₂ of the gas connecter 58 is relatively small, and therefore, the bending part 103 of the gas supplying and discharging pass 59 formed from the root end portion of the gas connecter 58 to the insertion instrument main part 53 is gentle, the conduit resistance in the bending part 103 is small, and the conduit resistance over the whole gas supplying and discharging pass 59 is small. Therefore, it is possible to supply the lubricant and supply to and discharge from air at the small resistance.

Moreover, the inner diameter ϕ₁ of the liquid supplying pass 57 is large and the conduit resistance thereof is small, and therefore, regarding the lubricant wherein the viscosity is relatively large and the resistance in supplying tends to be large, it is possible to supply the lubricant at the small resistance.

Furthermore, the inclination angle θ₁ of the liquid connecter 56 is large, and therefore, regarding the force applying to the liquid connecter 56 when inserting the endoscope 30 through the insertion instrument 52 and supplying the lubricant to the liquid connecter 56 of the insertion instrument 52, the component force in the longitudinal direction of the insertion instrument 52 is small, and the insertion instrument 52 is prevented from being displaced with respect to the endoscope 30.

In addition, the inclination angle θ₂ of the gas connecter 58 is small, and therefore, while the outer tube 62 is connected to the gas connecter 58, the inclination angle of the outer tube 62 is small and the insertion instrument 52 and the outer tube 62 have the integrated form along the longitudinal direction of the insertion instrument 52, whereby preventing the outer tube 62 from hampering an operation.

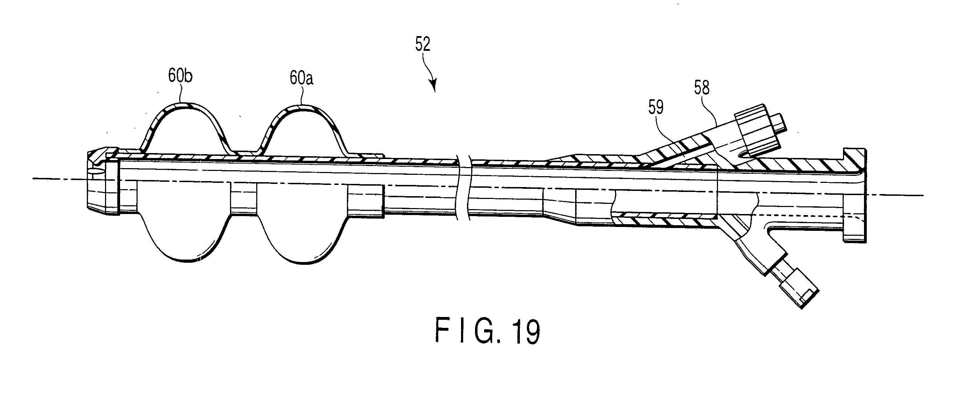

FIG. 19 shows a first variation example of the sixth embodiment of the present invention.

In an insertion instrument 52 according to the variation example, a first and a second balloon 60a, 60b are arranged side by side in the longitudinal direction of the insertion instrument 52 on the distal end portion of the insertion instrument 52. A gas supplying and discharging pass 59 is fluidly communicated with the first and the second balloon 60a, 60b.

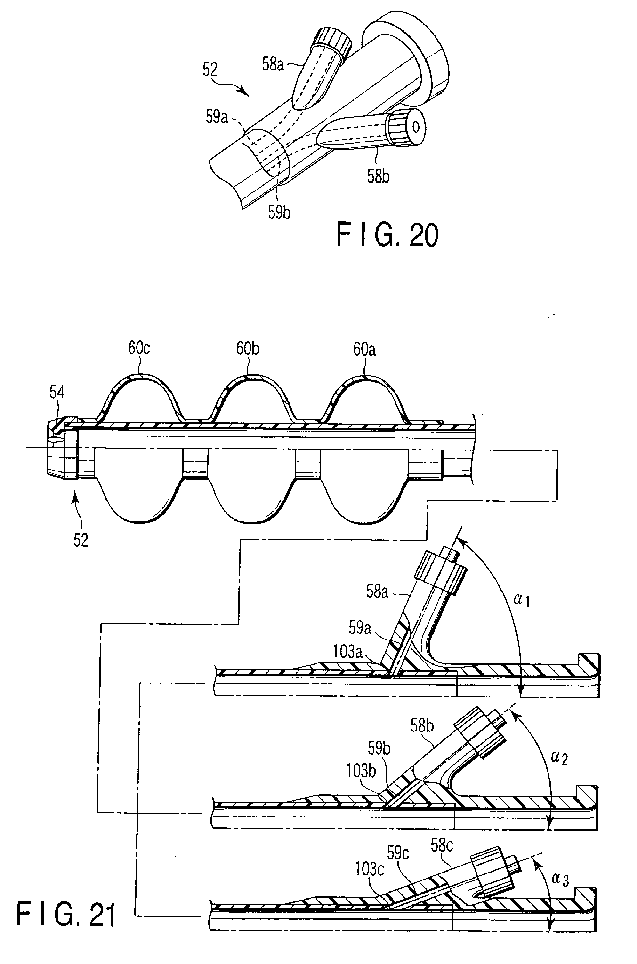

FIG. 20 shows a second variation example of the sixth embodiment of the present invention.

In an insertion instrument 52 according to the variation example, as is similar to the first variation example shown in FIG. 19, a first and a second balloon 60a, 60b is provided. A first and a second gas connecter 58a, 58b is provided on the proximal end portion of the insertion instrument 52, and the first and the second gas connecter 58a, 58b is fluidly communicated with the first and the second balloon 60a, 60b through a first and a second gas supplying and discharging pass 59a, 59b, respectively.

FIG. 21 shows a seventh embodiment of the present invention.

In the present embodiment, a first to a third balloon 60a, 60b, 60c is arranged side by side in the longitudinal direction of an insertion instrument 52 from the proximal end side to the distal end side on the distal end portion of the insertion instrument 52. A first to a third gas connecter 58a, 58b, 58c is provided on the proximal end portion of the insertion instrument 52, and the first to the third gas connecter 58a, 58b, 58c is fluidly communicated with the first to the third balloon 60a, 60b, 60c through a first to a third gas supplying and discharging pass 59a, 59b, 59c, respectively. Here, the first to the third gas connecter 58a, 58b, 58c forms a first to a third inclination angle α₁, α₂, α₃, and the second inclination angle α₂ is smaller than the first inclination angle α₁ and the third inclination angle α₃ is smaller than the second inclination angle α₂. That is, the inclination angle α₂, α₃ of the gas connecter 58b, 58c corresponding to the balloon 60b, 60c on more distal end side is smaller.

As is mentioned above, regarding the gas supplying and discharging pass 59b, 59c which extends to more distal end side, whose total length is larger and whose conduit resistance is larger amoung the gas supplying and discharging passes 59a, 59b, 59c corresponding to the balloons 60a, 60b, 60c, the inclination angle α₂, α₃ of the gas connecter 58b, 58c is smaller, the bending part 103b, 103c is gentler, and therefore, the inclination angles α₁, α₂, α₃ of the gas connecters 58a, 58b, 58c are optimally set.

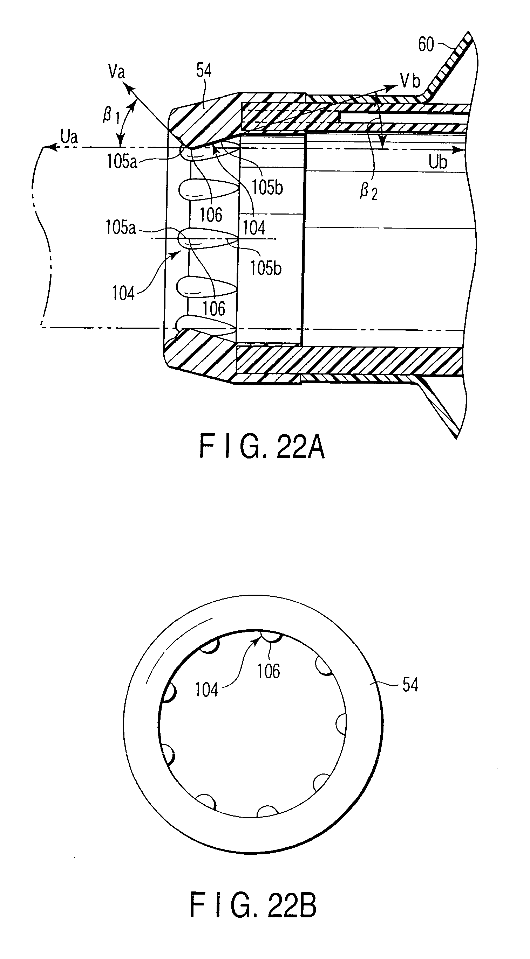

FIGS. 22A and 22B show a reference embodiment of the sixth and the seventh embodiment.

A distal end cap 54 is provided on the distal end portion of an insertion instrument main part 53, and protruding portions 104 extend in the longitudinal direction and are arranged side by side at certain intervals over the whole periphery on the inner surface of the distal end cap 54. In the protruding portion 104, an apex portion 106 is located on the center line extending in the longitudinal direction. In the transverse cross section cutting the protruding portion 104 perpendicularly to the center line, tapers are formed from the center to both the sides, respectively, and, in the longitudinal cross section cutting the protruding portion 104 along the center line, tapers 105a, 105b are formed from the apex portion to the distal end side and the proximal end side, respectively. A direction Va extending along the taper 105a from the apex portion 106 to the distal end side forms a first taper angle β₁ with respect to a longitudinal direction Ua toward the distal end side, and a direction Vb extending along the taper 105b from the apex portion 106 to the proximal end side forms a second taper angle β₂ with respect to a longitudinal direction Ub toward the distal end side. The first taper angle β₁ is larger than the second taper angle β₂.

From the perspective of reducing friction resistance in an insertion of the insertion portion 31 of the endoscope 30, it is preferable that the inner diameter of the distal end cap 54 of the insertion instrument 52 is sufficiently larger than the outer diameter of an insertion portion 31 of an endoscope 30. However, in the case where a large clearance is formed between the inner peripheral surface of the distal end cap 54 and the outer peripheral surface of the insertion portion 31, there is a possibility that a mucosa and the like is drawn into when drawing the insertion portion 31 into the distal end cap 54. In the reference embodiment, the only apex portion 106 of the protruding portion 104 of the distal end cap 54 is brought into contact with the insertion portion 31 when moving forward and backward the insertion portion 31 of the endoscope 30 with respect to the distal end cap 54, and therefore, friction resistance between the distal end cap 54 and the insertion portion 31 is sufficiently small. Moreover, when drawing the insertion portion 31 into the distal end cap 54, the protruding portion 104 prevents the mucosa from being drawn into. Furthermore, in the reference embodiment, the taper angle β₁ on the distal end side of the protruding portion 104 is relatively large, and the apex portion 106 is arranged on the relatively distal end side, and therefore, the mucosa is effectively prevented from being drawn into at the relatively distal end side of the distal end cap 54. Moreover, in the case where the taper 105a is not formed on the distal end side of the protruding portion 104, there is a possibility that unnecessary force applies to the mucosa while the mucosa is securely prevented from being drawn into, but such a situation is prevented in the reference embodiment.

Hereinafter, a eighth to a fourteenth embodiment of the present invention will be explained referring to the drawings.

The eighth to the fourteenth embodiment relates to an endoscope apparatus including an endoscope configured to be inserted into a cavity in the body and an insertion instrument adapted to assist an insertion of the endoscope into the cavity in the body.

When inserting an insertion portion of an endoscope into a cavity in the body, an insertion instrument adapted to assist an insertion of the insertion portion into the cavity in the body is used.

In the endoscope apparatus disclosed in Jpn. Pat. Appln. KOKAI Publication No. 2005-118115, an insertion portion of an endoscope is configured to be inserted through an insertion instrument main part having a shape of a sheath so as to be movable forward and backward. When inserting the insertion portion into a deep portion of a complexly bending cavity in the body, the insertion portion is moved forward with respect to the insertion instrument to be passed through a bending portion, and then, the insertion instrument is moved forward along the insertion portion to be passed through the bending portion, and the bending portion is kept in shape by the insertion instrument wherein an insertion can be easily carrid out, after that, the insertion portion is moved forward with respect to the insertion instrument. Furthermore, these operations are repeated. Here, balloons having a doughnut shape are provided on the distal end portion and the proximal end portion of the inner cavity of the insertion instrument coaxially with an insertion instrument main part, respectively. When the balloons are expanded and a liquid is filled into space closed tightly by the inner peripheral surface of the insertion instrument, both the balloons and the outer peripheral surface of the insertion portion to form a liquid layer, the insertion instrument and the insertion portion are scarcely brought into contact with each other, whereby reducing friction resistance between the insertion instrument and the insertion portion.

Moreover, in the endoscope apparatus, the insertion portion and an operation portion on the proximal end portion of the endoscope are coupled to each other through a coupling portion and the coupling portion has a larger diameter than that of the insertion portion. When the coupling portion is arranged at the position of the balloon on the proximal end portion of the insertion instrument and the balloon is expanded to tighten the coupling portion, the insertion instrument and the endoscope are fixed to each other.

In the endoscope apparatus disclosed in Jpn. Pat. Appln. KOKAI Publication No. 2005-118115, the insertion instrument and the endoscope are fixed to each other by expanding the balloon to tighten the insertion portion, and therefore, it is difficult to sufficiently fix the insertion instrument and the endoscope to each other.

Moreover, the balloon is provided in the inner cavity of the insertion instrument, and therefore, when inserting the endoscope into the insertion instrument, the balloon and the insertion portion are interfered with each other even when the balloon is contracted and it is impossible to smoothly carry out the insertion of the endoscope.

The eighth to the fourteenth embodiment has been made in view of the above mentioned problem and is directed to provide an endoscope apparatus wherein it is possible to sufficiently fix an endoscope and an insertion instrument to each other and smoothly insert the endoscope into the insertion instrument.

An endoscope apparatus and an insertion instrument according to the eighth to the fourteenth embodiment have similar structures to those of the endoscope apparatus according to the first embodiment as shown in FIGS. 1 and 2 and the insertion instrument according to the second embodiment explained referring to FIGS. 1 and 2. Hereinafter, configurations differing from the first and the second embodiment are explained in detail.

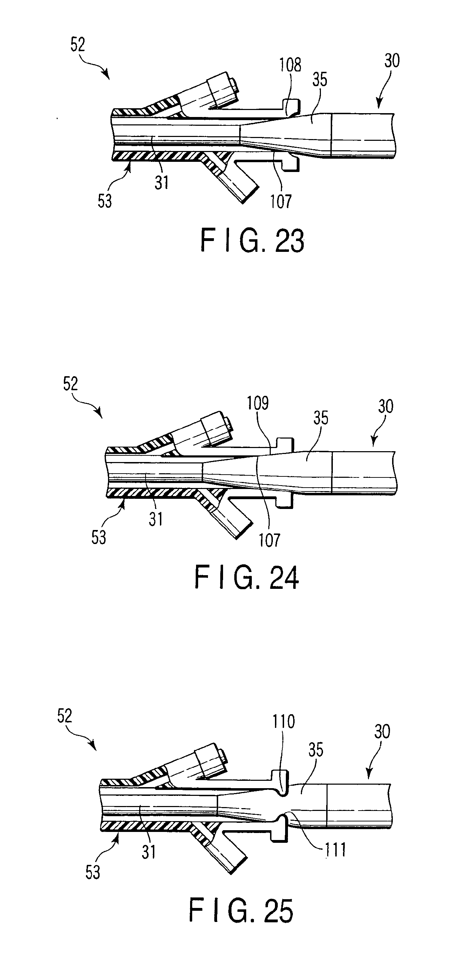

FIG. 23 shows the eighth embodiment of the present invention.

Referring to FIG. 23, a bending preventing portion 35 of an endoscope 30 has an outer diameter reducing from the proximal end side to the distal end side, and the outer peripheral surface 107 of the bending preventing portion 35 has a tapering shape. The inner diameter of an insertion instrument main part 53 of an insertion instrument 52 is larger than the outer diameter of an insertion portion 31 of the endoscope 30 or the outer diameter of the distal end portion of the bending preventing portion 35, and is smaller than the outer diameter of the proximal end portion of the bending preventing portion 35. Therefore, when the insertion portion 31 and then the bending preventing portion 35 are inserted into a proximal end opening of the insertion instrument main part 53 of the insertion instrument 52 and the bending preventing portion 35 is pressed into the insertion instrument main part 53, the outer peripheral surface 107 of the bending preventing portion 35 is fitted on an edge portion 108 of the proximal end opening of the insertion instrument main part 53. The diameter of the proximal end opening of the insertion instrument main part 53 of the insertion instrument 52 is larger than the outer diameter of the insertion portion 31, and therefore, the edge portion 108 of the proximal end opening is not fitted on the insertion portion 31.

That is, in the present embodiment, the outer peripheral surface 107 of the bending preventing portion 35 forms an endoscope fitting portion as an endoscope engagement portion, and the edge portion 108 of the proximal end opening of the insertion instrument main part 53 forms an insertion instrument fitting portion as an insertion instrument engagement portion.

Next, a method for using an endoscope apparatus according to the present embodiment will be explained.

The endoscope 30 is inserted through the insertion instrument 52, and the insertion instrument 52 and the endoscope 30 are fixed to each other to be integrated. That is, the insertion portion 31 of the endoscope 30 is inserted into a proximal end opening of the insertion instrument main part 53, and the insertion portion 31 is inserted through the inner cavity of the insertion instrument main part 53. In this time, the edge portion 108 of the proximal end opening of the insertion instrument main part 53 does not hamper the insertion of the insertion portion 31. Next, following the insertion portion 31, the bending preventing portion 35 is inserted into the proximal end opening of the insertion instrument main part 53, the bending preventing portion 35 is pressed into the insertion instrument main part 53, the outer peripheral surface 107 of the bending preventing portion 35 is fitted onto the edge portion 108 of the proximal end opening of the insertion instrument main part 53. In this way, the insertion instrument 52 and the endoscope 30 is fixed to each other.

The insertion instrument 52 and the endoscope 30 are inserted into a cavity in the body together with each other, and when the insertion instrument 52 and the endoscope 30 are reached to the shallow side of a bending part of the cavity in the body, the endoscope 30 is moved backward with respect to the insertion instrument 52, whereby releasing the fixing between the edge portion 108 of the proximal end opening of the insertion instrument main part 53 of the insertion instrument 52 and the outer peripheral surface 107 of the bending preventing portion 35 of the endoscope 30. In this way, the fixing between the insertion instrument 52 and the endoscope 30 is released.

A supplying and discharging apparatus 63 supplies a gas from the gas connecter 58 through the gas supplying and discharging pass 59 to the balloon 60, and the balloon 60 is expanded to hold the inner surface of the body wall. In this state, the insertion instrument 52 is moved backward, the body wall is hauled and the bending cavity in the body is made into a shape of a straight line, and then the endoscope 30 is moved forward with respect to the insertion instrument 52 to be passed through the parts of the cavity in the body, which has been bent. Then, when the endoscope 30 is moved forward to reach the shallow side of a next bending part of the cavity in the body, the supplying and discharging apparatus 63 discharge the gas from the balloon 60 of the insertion instrument 52, and the balloon 60 is contracted to release the holding to the inner surface of the body wall. Next, the insertion instrument 52 is moved forward along the endoscope 30 to the shallow side of the bending part, and the balloon 60 is expanded again to hold the inner surface of the body wall. Here, the cavity in the body is kept in the shape wherein the insertion can be easily carried out, within the range into which the insertion instrument 52 was inserted. After that, similar operations are repeated, and the endoscope 30 is inserted into the deep portion of the complexly bending cavity in the body. If necessary, the insertion instrument 52 and the endoscope 30 are fixed to each other and the fixing is released.

The endoscope apparatus according to the present embodiment exhibits the following effect.

In the endoscope apparatus according to the present embodiment, it is possible to sufficiently securely fix the endoscope 30 and the insertion instrument 52 to each other by fitting the outer peripheral surface 107 of the bending preventing portion 35 of the endoscope 30 and the edge portion 108 of the proximal end opening of the insertion instrument main part 53 of the insertion instrument 52. Moreover, it is impossible to fit the edge portion 108 of the proximal end opening of the insertion instrument main part 53 on the insertion portion 31 of the endoscope 30, and therefore, the edge portion 108 scarcely hampers an insertion when inserting the insertion portion 31 into the insertion instrument 52, and it is possible to smoothly insert the insertion portion 31 into the insertion instrument 52.

FIG. 24 shows the ninth embodiment of the present invention.

In an insertion instrument 52 of an endoscope apparatus according to the present embodiment, a tapering portion 109 is formed on the inner peripheral surface side of the proximal end portion of an insertion instrument main part 53. The tapering portion 109 has an inner diameter reducing from the proximal end side to the distal end side, and corresponds to a tapering shape of an outer peripheral surface 107 of a bending preventing portion 35 of an endoscope 30. Therefore, when an insertion portion 31 and then the bending preventing portion 35 are inserted into the proximal end opening of the insertion instrument main part 53, the outer peripheral surface 107 of the bending preventing portion 35 is fitted on the tapering portion 109 of the proximal end portion of the insertion instrument main part 53. Moreover, the inner diameter of the distal end portion of the tapering portion 109 is larger than the outer diameter of the insertion portion 31 of the endoscope 30, and the tapering portion 109 does not fit on the insertion portion 31.

A method for using the endoscope apparatus according to the present embodiment is similar to the method for using the endoscope apparatus according to the eighth embodiment. That is, when fixing the insertion instrument 52 and the endoscope 30 to each other, the insertion portion 31 and then the bending preventing portion 35 is inserted into the proximal end opening of the insertion instrument main part 53 of the insertion instrument 52, and the outer peripheral surface 107 of the bending preventing portion 35 is fitted onto the tapering portion 109 of the proximal end portion of the insertion instrument main part 53. Here, the tapering portion 109 of the proximal end portion of the insertion instrument main part 53 does not hamper an insertion of the insertion portion 31.

In the endoscope apparatus according to the present embodiment, the outer peripheral surface 107 of the bending preventing portion 35 which is generally used in the endoscope 30 is utilized for fitting with the insertion instrument 52, and therefore, it is unnecessary to additionally process the endoscope 30, and it is possible to inexpensively embody.

Moreover, the tapering portion 109 of the proximal end portion of the insertion instrument main part 53 of the insertion instrument 52 and the outer peripheral surface 107 of the bending preventing portion 35 of the endoscope 30 are fitted on each other, and therefore, it is possible to securely fix the insertion instrument 52 and the endoscope 30 to each other in comparison with the endoscope apparatus according to the eighth embodiment.

FIG. 25 shows the tenth embodiment of the present invention.

In an endoscope apparatus according to the present embodiment, a fitting convex portion 110 is formed on the inner peripheral surface side of the proximal end portion of an insertion instrument main part 53 of an insertion instrument 52 and protrudes radially inwardly. On the other hand, a fitting concave portion 111 is formed on the outer peripheral surface portion of a bending preventing portion 35 of an endoscope 30, depresses radially inwardly and corresponds to the fitting convex portion 110. Therefore, when the insertion portion 31 and then the bending preventing portion 3 are inserted into a proximal end opening of the insertion instrument main part 53, the fitting convex portion 110 of the proximal end portion of the insertion instrument main part 53 is fitted on the fitting concave portion 111 of the bending preventing portion 35. Moreover, the inner diameter at the radially inwardly end portion of the fitting convex portion 110 is larger than the outer diameter of the insertion portion 31 of the endoscope 30, and the fitting convex portion 110 is not fitted on the insertion portion 31.

A method for using the endoscope apparatus according to the present embodiment is similar to the method for using the endoscope apparatus according to the eighth embodiment. That is, when fixing the insertion instrument 52 and the endoscope 30 to each other, the insertion portion 31 and then the bending preventing portion 35 are inserted into the proximal end opening of the insertion instrument main part 53 of the insertion instrument 52, and the fitting convex portion 110 of the proximal end portion of the insertion instrument main part 53 is fitted on the fitting concave portion 111 on the outer peripheral portion of the bending preventing portion 35. Here, the fitting convex portion 110 of the proximal end portion of the insertion instrument main part 53 does not hamper an insertion of the insertion portion 31.

In the endoscope apparatus according to the present embodiment, the fitting convex portion 110 of the proximal end portion of the insertion instrument main part 53 of the insertion instrument 52 and the fitting concave portion 111 of the outer peripheral portion of the bending preventing portion 35 of the endoscope 30 are fitted on each other, and therefore, it is possible to further securely fix the insertion instrument 52 and the endoscope 30 in comparison with the endoscope apparatus according to the ninth embodiment.

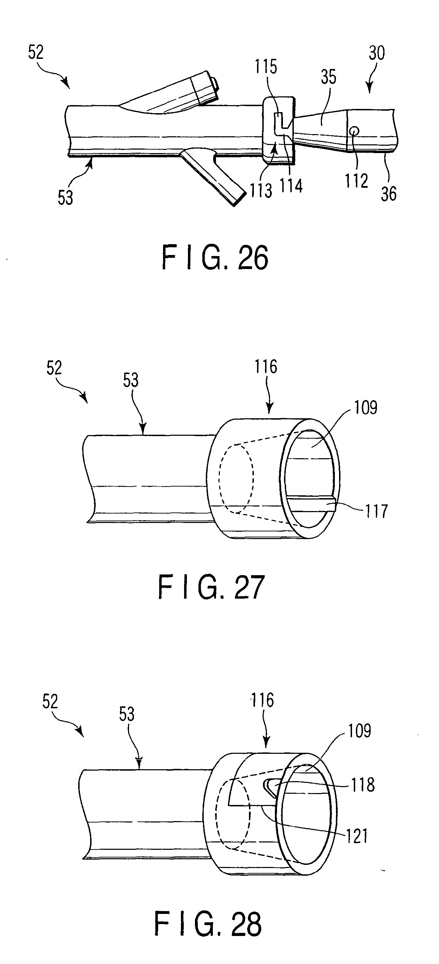

FIG. 26 shows the eleventh embodiment of the present invention.

In an endoscope apparatus according to the present embodiment, the inner diameter of an insertion instrument main part 53 of an insertion instrument 52 is larger than the outer diameters of an insertion portion 31 of an endoscope 30, a bending preventing portion 35 and the distal end portion of an operation portion 36. A pin 112 protrudes radially outwardly from the distal end portion of the operation portion 36 of the endoscope 30. On the other hand, an engagement groove portion 113 having a shape of a through groove is formed on the proximal end portion of the insertion instrument main part 53 of the insertion instrument 52, and the pin 112 is slidable in the engagement groove portion 113. In the engagement groove portion 113, a guide portion 114 configured to guide the pin 112 extends in the longitudinal direction from the proximal end of the insertion instrument main part 53 and then a holding portion 115 configured to hold the pin 112 extends in the peripheral direction.

A method for using the endoscope apparatus according to the present embodiment is similar to the method for using the endoscope apparatus according to the eighth embodiment. However, when fixing the insertion instrument 52 and the endoscope 30 to each other, after the insertion portion 31 and the bending preventing portion 35 are inserted into a proximal end opening of the insertion instrument main part 53, the insertion instrument 52 is rotated with respect to the endoscope 30 and the pin 112 on the distal end portion of the operation portion 36 of the endoscope 30 is positioned to the inlet of the engagement groove portion 113. Then, the endoscope 30 is moved forward with respect to the insertion instrument 52 and the pin 112 is slid along the guide portion 114 of the engagement groove portion 113, and then, the endoscope 30 is rotated with respect to the insertion instrument 52 and the pin 112 is put into and held in the holding portion 115 of the engagement groove portion 113. As a result, the endoscope 30 is fixed with respect to the insertion instrument 52 so as to be unmovable forward and backward. When releasing the fixing between the insertion instrument 52 and the endoscope 30, the endoscope 30 is rotated with respect to the insertion instrument 52 and the pin 112 is put into the guide portion 114 from the holding portion 115 of the engagement groove portion 113, and then, the endoscope 30 is moved backward with respect to the insertion instrument 52 and the pin 112 is slid along the guide portion 114 of the engagement groove portion 113 and pulled out the engagement groove portion 113.

In the endoscope apparatus according to the present embodiment, it is possible to securely fix the endoscope 30 with respect to the insertion instrument 52 so as to be unmovable forward and backward by holding the pin 112 in the holding portion 115 of the engagement groove portion 113. Moreover, the insertion instrument 52 and the endoscope 30 is fixed and the fixing is released by moving forward and backward and rotating the endoscope 30 with respect to the insertion instrument 52 to guide the pin 112 to and form the holding portion 115 through the guide portion 114 of the engagement groove portion 113, and therefore, it is possible to easily fix and release at a relatively small amount of operation force in comparison with the case where the fixing is carried out by fitting.

FIG. 27 shows the twelfth embodiment of the present invention.

In the case where an insertion instrument 52 and an endoscope 30 are fixed to each other through fitting as the ninth embodiment shown in FIG. 24, there is a possibility that a fitting surface of the insertion instrument 52 and a fitting surface of the endoscope 30 are adhered to each other when they are kept fitted for a long time. For example, in the case where both the fitting surfaces are made of material which tends to adhere such as silicon, when humor and so on interposed between both the fitting surfaces is dried and hardened, there is a possibility of adhesion.

Referring to FIG. 27, in the present embodiment, an adhesion preventing mechanism adapted to prevent such adhesion is formed on an insertion instrument fitting portion. That is, a large diameter portion 116 having a large outer diameter is formed on the proximal end portion of the insertion instrument 52, and a tapering portion 109 similar to that according to the eleventh embodiment is formed on the inner peripheral surface side of the large diameter portion 116. In the tapering portion 109, an adhesion preventing groove portion 117 extends in the longitudinal direction.

In an endoscope apparatus of the present embodiment, the adhesion preventing groove portion 117 prevents an outer peripheral surface 107 of a bending preventing portion 35 of an endoscope 30 and the tapering portion 109 of the insertion instrument 52 from being adhered to each other, and therefore, it is prevented that releasing the fixing between the insertion instrument 52 and the endoscope 30 is made impossible.

FIG. 28 shows the thirteen embodiment of the present invention.

In the present embodiment, an adhesion releasing mechanism adapted to release adhesion between a fitting surface of an insertion instrument 52 and a fitting surface of an endoscope 30 is formed on an insertion instrument fitting portion. That is, perforations 121 is formed on the peripheral wall of a large diameter portion 116 of the proximal end portion of an insertion instrument 52, similar to that according to the twelfth embodiment. The perforations 121 extend from the proximal end of the large diameter portion 116 in the longitudinal direction and then extend in the peripheral direction. A grasping portion 118 is formed on the region surrounded by the perforations 121 on the proximal end of the large diameter portion 116. When the tapering portion 109 of the large diameter portion 116 of the insertion instrument 52 and an outer peripheral surface 107 of a bending preventing portion 35 of an endoscope 30 are adhered to each other, the grasping portion 118 of the large diameter portion 116 is grasped and pulled up, and the peripheral wall of the large diameter portion 116 is tear along the perforations 121 while cutting the perforations 121, whereby releasing the adhesion between both the fitting surfaces.

That is, in the present embodiment, a breaking mechanism adapted to break the large diameter portion 116 forms an adhesion releasing mechanism. It is noted that the large diameter portion 116 may be configured to be attached to and detached from an insertion instrument main part 53 and the broken large diameter portion 116 may be exchanged for a new large diameter portion 116.

In an endoscope apparatus according to the present embodiment, it is possible to release adhesion between the tapering portion 109 of the large diameter portion 116 of the insertion instrument 52 and the outer peripheral surface 107 of the bending preventing portion 35 of the endoscope 30 by breaking the large diameter portion 116 of the proximal end portion of the insertion portion 31, and therefore, it is possible to easily release the fixing between the insertion instrument 52 and the endoscope 30 even when the tapering portion 109 and the outer peripheral surface 107 are adhered.

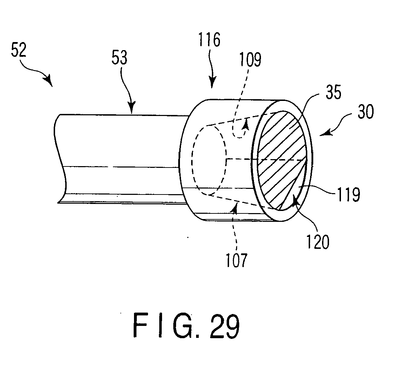

FIG. 29 shows the fourteenth embodiment of the present invention.

In the present embodiment, a plane surface portion 119 is formed on a bending preventing portion 35 of an endoscope 30 and is configured not to be brought into contact with a tapering portion 109 of an insertion instrument 52. Therefore, when an outer peripheral surface 107 of the bending preventing portion 35 is fitted on the tapering portion 109, deformation space is formed between the tapering portion 109 and the plane surface portion 119 of the bending preventing portion 35 and the peripheral wall of a large diameter portion 116 is radially inwardly deformable into the deformation space. In the case where the tapering portion 109 and the outer peripheral surface 107 of the bending preventing portion 35 are adhered to each other, the peripheral wall of the large diameter portion 116 is radially inwardly deformed into the deformation space and the whole peripheral wall of the large diameter portion 116 is deformed into a shape of an ellipse, and the tapering portion 109 and the outer peripheral surface 107 of the bending preventing portion 35 are separated form each other, whereby releasing the adhesion. That is, in the present embodiment, a deformation mechanism adapted to deform the large diameter portion 116 forms an adhesion releasing mechanism.

In an endoscope apparatus according to the present embodiment, adhesion is released by deforming the large diameter portion 116 to separate the tapering portion 109 and the outer peripheral surface 107 of the bending preventing portion 35 from each other, and therefore, it is possible to easily and inexpensively release the fixing in comparison with the case where the large diameter portion 116 is broken as the seventh embodiment.

## Claims

1. A medical apparatus **characterized by** comprising
a hollow member (53; 60; 62; 64; 98) whose inner surface parts are configured to be brought into contact with each other through deformation of the hollow member (53; 60; 62; 64; 98), and
wherein the hollow member (53; 60; 62; 64; 98) includes a convex and concave portion (70) formed on the inner surface parts configured to be brought into contact with each other.

2. The medical apparatus according to claim 1, **characterized in that**
the hollow member (53; 60; 62; 64; 98) is formed of an elastic member.

3. The medical apparatus according to claim 2, **characterized in that**
the elastic member is made of silicon, polyurethane, or vinyl chloride.

4. The medical apparatus according to claim 1, **characterized in that**
the hollow member (60) is a balloon (60) configured to be expanded and contracted.

5. The medical apparatus according to claim 1, **characterized in that**
the hollow member (62; 64) is a fluid transferring tube (62; 64).

6. The medical apparatus according to claim 5, **characterized in that**
the fluid transferring tube (64) is configured to be closed and opened by a valve configured to press the fluid transferring tube (64) from an outside.

7. The medical apparatus according to claim 5, **characterized in that**
the fluid transferring tube (64) is housed within the medical apparatus.

8. The medical apparatus according to claim 5, **characterized in that**
a contamination generating source is located on an upstream side and the fluid transferring tube (64) is provided on more downstream side than a contamination removing mechanism (92) with respect to the contamination generating source.

9. The medical apparatus according to claim 1, **characterized in that**
the hollow member (53) is an insertion instrument main part (53) for an endoscope including an inner cavity through which an insertion portion (31) of an endoscope (30) is inserted so as to be movable forward and backward.

10. The medical apparatus according to claim 9, **characterized by** comprising:
the insertion instrument main part (53) having a tubular shape and including a distal end portion and a proximal end portion;
a liquid transferring pass (57) extending from the proximal end portion of the insertion instrument main part (53) toward a distal end side and configured to transfer a liquid;
a gas transferring pass (59; 59a) extending from the proximal end portion of the insertion instrument main part (53) toward the distal end side and configured to transfer a gas;
a liquid connecter (56) protruding from the proximal end portion of the insertion instrument main part (53), forming a proximal end portion of the liquid transferring pass (57), configured to be connected to a liquid transferring apparatus (51) and whose protruding direction (R) and a longitudinal direction (P) of the insertion instrument main part (53) toward a distal end side forms a first inclination angle (θ₁); and
a gas connecter (58; 58a) protruding from the proximal end portion of the insertion instrument main part (53), forming a proximal end portion of the gas transferring pass (59; 59a), configured to be connected to a gas transferring apparatus (63) and whose protruding direction (T) and the longitudinal direction (P) of the insertion instrument main part (53) toward the distal end side forms a second inclination angle (θ₂; α₁), and
wherein the second inclination angle (θ₂; α₁) is smaller than the first inclination angle (θ₁).

11. The medical apparatus according to claim 10, **characterized in that**
an inner diameter of the liquid transferring pass (57) is larger than an inner diameter of the gas transferring pass (59; 59a).

12. The medical apparatus according to claim 10 or 11, **characterized in that**
the liquid transferring pass (57) is configured to transfer a lubricant.

13. The medical apparatus according to claim 10, **characterized in that**
the liquid transferring pass (57) is a liquid supplying pass (57) configured to supply a liquid.

14. The medical apparatus according to claim 10, **characterized in that**
a gas tube (62) is to be connected to the gas connecter (58; 58a) and the gas connecter (58; 58a) and the gas transferring apparatus (63) are to be connected to each other through the gas tube (62).

15. The medical apparatus according to claim 10, **characterized by** comprising:
other gas transferring pass (59b) extending from the proximal end portion of the insertion instrument main part (53) to more distal end side of the insertion instrument main part (53) than the gas transferring pass (59a) and configured to transfer a gas; and
other gas connecter (58b) protruding from the proximal end portion of the insertion instrument main part (53), forming a proximal end portion of the other gas transferring pass (59b), configured to be connected to a gas transferring apparatus (63) and whose protruding direction (T) and the longitudinal direction (P) of the insertion instrument main part (53) toward the distal end side forms other second inclination angle (α₂), and
wherein the other second inclination angle (α₂) is smaller than the second inclination angle (α₁).

16. The medical apparatus according to claim 9, **characterized by** comprising:
an endoscope (30) including an insertion portion (31) provided on a distal end side of the endoscope (30) and configured to be inserted into a cavity in a body and an endoscope engagement portion (107; 111; 112) provided in a proximal end portion of the endoscope (30);
an insertion instrument (52) including the insertion instrument main part (53) with a distal end portion and a proximal end portion and an insertion instrument engagement portion (108; 110; 113) provided in the proximal end portion of the insertion instrument main part (53) and configured to be engaged with the endoscope engagement portion (107; 111; 112).

17. The medical apparatus according to claim 16, **characterized in that**
the endoscope engagement portion (107; 111) and the insertion instrument engagement portion (108; 110) include an endoscope fitting portion (107; 111) and an insertion instrument fitting portion (108; 110) configured to be fitted to each other, respectively.

18. The medical apparatus according to claim 17, **characterized in that**
the endoscope (30) includes a bending preventing portion (35) provided on more proximal end side than the insertion portion (31) and whose outer peripheral surface (107) has a tapering shape,
the endoscope fitting portion (107) is formed of the outer peripheral surface (107) of the bending preventing portion (35), and
the insertion portion fitting portion (109) includes a tapering portion (109) configured to be fitted to the outer peripheral surface of the bending preventing portion (35).

19. The medical apparatus according to claim 17, **characterized in that**
one fitting portion (110) of the endoscope fitting portion (111) and the insertion instrument fitting portion (110) includes a fitting convex portion (110), and
the other fitting portion (111) of the endoscope fitting portion (111) and the insertion instrument fitting portion (110) includes a fitting concave portion (111) configured to be fitted to a fitting convex portion (110).

20. The medical apparatus according to claim 16, **characterized in that**
one engagement portion (112) of the endoscope engagement portion (112) and the insertion instrument engagement portion (113) includes a pin (112),
the other engagement portion (113) of the endoscope engagement portion (112) and the insertion instrument engagement portion (113) includes an engagement groove portion (113) in which the pin (112) is configured to be slid, and
the engagement groove portion (113) includes a holding portion (115) configured to hold the pin (112) such that the endoscope (30) is unmovable forward and backward with respect to the insertion instrument (52) and a guide portion (114) connecting to the holding portion (115) and configured to guide the pin (112).

21. The medical apparatus according to claim 17, **characterized in that**
at least one fitting portion (109) of the endoscope fitting portion (107) and the insertion instrument fitting portion (109) includes an adhesion preventing mechanism (117) configured to prevent adhesion between the endoscope fitting portion (107) and the insertion instrument fitting portion (109).

22. The medical apparatus according to claim 21, **characterized in that**
the adhesion preventing mechanism (117) includes an adhesion preventing concave portion (117) formed on a fitting surface of the at least one fitting portion (109) of the endoscope fitting portion (107) and the insertion instrument fitting portion (109).

23. The medical apparatus according to claim 17, **characterized in that**
at least one fitting portion (109) of the endoscope fitting portion (107) and the insertion instrument fitting portion (109) includes an adhesion releasing mechanism (118, 121; 119, 120) configured to release adhesion between the endoscope fitting portion (107) and the insertion instrument fitting portion (109).

24. The medical apparatus according to claim 23, **characterized in that**
the adhesion releasing mechanism (118, 121) is formed of a breaking mechanism (118, 121) configured to break the at least one fitting portion (109) of the endoscope fitting portion (107) and the insertion instrument fitting portion (109).

25. The medical apparatus according to claim 23, **characterized in that**
the adhesion releasing mechanism (119, 120) is formed of a deformation mechanism (119, 120) configured to deform the at least one fitting portion (109) of the endoscope fitting portion (107) and the insertion instrument fitting portion (109) to separate a fitting surface of the endoscope fitting portion (107) and a fitting surface of the insertion instrument fitting portion (109) from each other.

26. The endoscope of the medical apparatus according to any one of claims 16 to 25.

27. The insertion instrument of the medical apparatus according to any one of claims 16 to 25.
